# EUROPÄISCHE PATENTSCHRIFT

(11) **EP 0 769 490 B1**
(45) Veröffentlichungstag und Bekanntmachung des Hinweises auf die Patenterteilung: **19.12.2001**
(21) Anmeldenummer: 96116773.1
(22) Anmeldetag: 18.10.1996
(51) Int. Cl.: C07C 245/08, C07D 495/04, C07D 333/00, G01N 33/53

(54) **Reagenz zum Nachweis und zur Isolierung von Kohlehydraten oder Glycanrezeptoren**
Reagent for the detection and isolation of carbohydrates or glycan-receptors
Réactifs pour l'identification et l'isolation des carbohydrates ou des récepteurs glycaniques

(30) Priorität: 19.10.1995 DE 19539008
(43) Veröffentlichungstag der Anmeldung: 23.04.1997
(73) Patentinhaber: Roche Diagnostics GmbH, 68305 Mannheim (DE)
(72) Erfinder: Watzele, Manfred, Dr., 82362 Weilheim (DE); Fernholz, Erhard, Dr., 82362 Weilheim (DE); Von der Eltz, Herbert, Dr., 82362 Weilheim (DE)
(74) Vertreter: Weiss, Wolfgang, Dipl.-Chem. Dr.

(56) Entgegenhaltungen:
- WO-A-87/04794
- DE-A- 3 629 194
- US-A- 5 252 743
- PATENT ABSTRACTS OF JAPAN vol. 16, no. 586 (P-1463), 25.Dezember 1992 & JP 04 236353 A (SHIN ETSU CHEM. CO. LTD.)
- THE JOURNAL OF BIOLOGICAL CHEMISTRY, Bd. 256, Nr. 2, 1981, Seiten 761-766, XP002024148 G. A. ORR: "The Use of the 2-Iminobiotin-Avidin Interaction for the Selective Retrieval of Labeled Plasma Membrane Components"
- ANAL. CHEM., Bd. 68, 1.August 1996, Seite 2573-2579 XP000614745 Y. SHINOHARA ET AL.: "Bifunctional Labeling Reagent for Oligosaccharides to Incorporate Both Chromophore and Biotin Groups"

## Beschreibung

Die Erfindung betrifft Verbindungen, die einen Chromophor und eine an Streptavidin oder/und Avidin bindefähige Gruppe aufweisen und geeignet sind zur Anbindung an Moleküle, die eine Aldehyd-, Keton-, Hemiacetal- oder/und Hemiketalfunktion enthalten. Weiterhin betrifft die Erfindung aus diesen Verbindungen gebildete Konjugate, sowie ein Verfahren zum Nachweis, bzw. zur Isolierung von Kohlehydraten oder Glycanrezeptoren mittels solcher Konjugate.

Glykokonjugate sind in vielen biologischen Bereichen, beispielsweise als Enzyme, Transportproteine, Rezeptorproteine, Hormone oder Strukturproteine anzutreffen. Die Wechselwirkungen von freien Sacchariden sowie von Saccharidanteilen der Glykokonjugate mit spezifischen Rezeptoren spielen eine wichtige Rolle bei der biologischen Funktion dieser Verbindungen.

Um diese Wechselwirkungen untersuchen zu können, müssen die entsprechenden Saccharide in möglichst reiner Form erhalten werden. Aufgrund der großen Vielfalt in der Saccharide in natürlichen Proben vorliegen, sowie der strukturellen Ähnlichkeit vieler Saccharide, ist eine Isolierung von reinen definierten Sacchariden jedoch schwierig. Insbesondere die geringe UV-Sensitivität von Sacchariden erschwert die Detektion von geringen Mengen.

Es sind verschiedene Verfahren bekannt, die zur Fraktionierung und Detektion von Sacchariden eingesetzt werden. Die Analyse mittels High pH Anion Exchange (HPAE)-Chromatographie mit gepulster amperometrischer Detektion (PAD) erlaubt die direkte Quantifizierung von Sacchariden bei einfacher Probenvorbereitung, ohne daß eine Derivatisierung notwendig ist. Der Nachteil dieses Verfahrens besteht allerdings darin, daß die Saccharide aufgrund der stark basischen Bedingungen einerseits in Mitleidenschaft gezogen und andererseits nicht direkt in Bindungsstudien eingesetzt werden können.

Bei einem weiteren Verfahren werden die Saccharide zunächst derivatisiert. Dazu werden die Saccharide mit UV- oder fluoreszenzaktiven Reagenzien markiert, wie z.B. durch Derivatisierung mit 2-Aminopyridin (Rice et al., Anal. Biochem. 206 (1992), 278 - 287). Die Nachteile des dort beschriebenen Verfahrens, liegen in den hohen Umsetzungstemperaturen, die zur Zersetzung der Saccharide führen können (Kakehi et al., Anal. Biochem. 199 (1991), 256) sowie in der Verwendung toxischer Reagenzien wie z.B. NaCNBH₃. Darüberhinaus lassen sich die erhaltenen Konjugate nicht ohne weiteres in Bindungsstudien einsetzen.

Auch Verfahren zur Charakterisierung von Glycan-Rezeptor-Wechselwirkungen sind beschrieben worden. Lösliche Saccharide werden dabei so derivatisiert, so daß sie an eine Festphase oder an Makromoleküle gebunden werden können. Dadurch wird eine Untersuchung der Wechselwirkungen zwischen Rezeptor und Saccharid möglich. Beispielsweise werden bei dem von Feizi et al. beschriebenen Verfahren (Feizi et al., Methods Enzymol. 230 (1994), 484 - 519) die Saccharide mit einem Lipid derivatisiert und auf Dünnschichtplatten aufgetrennt. Anschließend werden direkt auf der Platte die Bindungsstudien durchgeführt. Nachteilig bei diesem Verfahren ist jedoch die geringe Sensitivität der Anfärbung und Detektion des Saccharids. Zudem ist die Auftrennung auf ein einziges Trennprinzip, nämlich die Dünnschichtchromatographie, beschränkt. Außerdem kann die Derivatisierung durch Kopplung von Oligosacchariden an Neoglycoproteine (Hubert et al., Cell. Diff. Dev. 27 (1989), 69) oder direkt an Biotin erfolgen. Die so erhaltenen Derivate sind jedoch für eine herkömmliche Fraktionierung und Detektion ungeeignet, so daß erst andere Wege zu deren Reinigung gefunden werden müssen.

Der Versuch, mit einem einzigen Reagenz die Detektion und Fraktionierung von Sacchariden, sowie deren direkte Charakterisierung in Bindungsstudien zu ermöglichen, wurde von Toomre und Varki mit der Darstellung und Anwendung des Reagenz 2-Amino-6-amidobiotinyl-pyridin unternommen (Toomre et al., Glycobiol. 4 (1994), 653 - 663). Es wird dabei ein Konjugat aus Saccharid und dem eine Amin-Funktion enthaltenden Reagenz gebildet. Das Reagenz weist neben der Amin-Funktion die fluoreszierenden Eigenschaften des 2-Aminopyridin und die Affinität von Biotin an Streptavidin auf. Dadurch konnten nach sensitiver Auftrennung direkt Bindungsstudien durchgeführt werden. Nachteilig an diesem Verfahren sind jedoch die je nach Saccharid unterschiedlichen Kopplungseffizienzen, die der Patentanmeldung WO94/28008 von Varki und Hotenberg zu entnehmen sind, die mögliche Zersetzung der Saccharide unter den Reaktionsbedingungen und die Toxizität des Reduktionsmittels.

Darüber hinaus liegt im Konjugat durch den Schritt der reduktiven Aminierung der Zucker am reduzierenden Ende stets in der offenkettigen Form vor und ist nicht mehr in der Lage, eine geschlossene Ringstruktur zu bilden. Durch die Bildung des Konjugats wird somit die Struktur des Saccharids verändert. Da aber gerade das Saccharid die zu untersuchende Erkennungsstruktur darstellt, kann der für das Saccharid spezifische Rezeptor unter Umständen nicht an das Saccharid binden, was einen wesentlichen Nachteil dieses Verfahrens darstellt.

Nachweisreagenzien die zur Untersuchung der Wechselwirkungen von Sacchariden mit Rezeptoren geeignet sind, sollten idealerweise folgende Eigenschaften aufweisen:
- das Nachweisreagenz soll eine Markierungsgruppe enthalten, mittels derer die Saccharide durch UV/VIS oder Fluoreszenzspektrometrie mit hoher Sensitivität detektiert werden können,
- das durch Kopplung des Saccharids mit dem Nachweisreagenz entstehende Konjugat soll an Makromoleküle oder Festphasen bindefähig sein: diese Eigenschaft kann wahlweise zur Immobilisierung oder zur Detektion des Saccharids verwendet werden,
- die Saccharide sollen unter den Reaktionsbedingungen nicht zersetzt werden,
- bei der Derivatisierung soll eine geschlossene Ringstruktur am reduzierenden Ende des Zuckers möglich sein,
- derivatisierte Saccharidgemische sollen auf einfache Weise aufgetrennt werden können,
- das bei der Derivatisierung üblicherweise im Überschuß eingesetzte Nachweisreagenz soll sich relativ einfach von den umgesetzten Sacchariden abtrennen lassen,
- die zur Derivatisierung benötigten Reagenzien sollen möglichst geringe oder keine Toxizität zeigen,
- die Effizienz der Kopplungsreaktion soll für alle Saccharide hoch sein,
- das Nachweisreagenz soll eine Analyse mittels Massenspektrometrie erleichtern,
- das Nachweisreagenz soll die Wechselwirkung zwischen dem Saccharid und dem Liganden nicht beeinflussen.

Eine Aufgabe der vorliegenden Erfindung war es somit, verbesserte Reagenzien zum Nachweis und zur Isolierung von Sacchariden bzw. von Glycanrezeptoren bereitzustellen.

Die Aufgabe wird erfindungsgemäß gelöst durch eine Verbindung mit einer Struktur der Formel (I):

X - Y (I)

worin X einen Rest darstellt, ausgewählt aus -NR-NH₂, -C(A)-NR-NH2 oder -B-C(A)-NR-NH₂, wobei R Wasserstoff oder ein Alkyrest mit 1 bis 4 C-Atomen ist und A und B jeweils unabhängig NH, O oder S darstellen und Y einen Rest darstellt, der einen fluoreszierenden Chromophor oder einen Chromophor, welcher ein Absorptionsmaximum bei einer Wellelänge λₘₐₓ von mehr als 250 nm oder/und bei der Wellenlänge λₘₐₓ des Absorptionsmaximums einen molaren Extinktionskoeffizienten e von mehr als 1000 1 mol⁻¹ cm⁻¹ aufweist sowie einen an Streptavidin oder/und Avidin bindefähigen Liganden enthält.

Eine solche Verbindung kann über den Rest X an Keton-, Aldehyd-, Semiketal- oder Semiacetal-Gruppen gekoppelt werden, die insbesondere in Sacchariden vorliegen. Die resultierenden Konjugate besitzen eine chromophore Gruppe und sind an Streptavidin bzw. Avidin bindefähig. Die Verbindung (I) besitzt vorzugsweise im UV/VIS-Bereich einen hohen Extinktionswert ∈ und ermöglicht somit eine sensitive Detektion der nachzuweisenden Substanz. Weiterhin kann durch den an Avidin oder/und Streptavidin bindefähigen Liganden eine Kopplung der zu untersuchenden Substanz an eine Festphase oder ein Makromolekül erhalten werden. Wie im folgenden näher erläutert, kann die Verbindung (I) unter Reaktionsbedingungen an Saccharide geknüpft werden, bei denen die Saccharide nicht zersetzt werden. Weiterhin werden keine toxischen Reagenzien zur Umsetzung benötigt. Die Verbindung (I) reagiert selektiv mit dem reduzierenden Ende von Sacchariden, wobei deren Fähigkeit zur Ausbildung einer geschlossenen Ringstruktur erhalten bleibt. Außerdem kann im Überschuß eingesetztes Nachweisreagenz einfach mittels Phasentrennung von den erhaltenen Konjugaten abgetrennt werden.

X kann einen Rest -NR-NH₂ darstellen, der an eine Alkylen-, Alkenylen- oder Alkinylengruppe oder an eine aromatische Gruppe gebunden ist, -beispielsweise ein Hydrazin. Weiterhin kann X die Gruppe -B-C(A)-NR-NH₂ darstellen, worin A, B und R wie oben definiert sind, beispielsweise ein Semicarbazid, oder die Gruppe -C(A)-NR-NH₂, beispielsweise ein Hydrazid.

Erfindungsgemäß weist der Chromophor, der im Rest Y enthalten ist, ein Absorptionsmaximum bei einer Wellenlänge λₘₐₓ von mehr als 250 nm auf. Dadurch kann eine sensitive Detektion in herkömmlichen UV/VIS-Spektrometern erfolgen. Besitzt der Chromophor auch fluorophore Eigenschaften, so ist auch eine Detektion mit Fluoreszenzdetektoren möglich. Der Chromophor weist zudem bei der Wellenlänge λₘₐₓ des Absorptionsmaximums einen molaren Extinktionskoeffizienten ∈ von mehr als 1000 l mol⁻¹cm⁻¹ und bevorzugt von mehr als 3000 l mol⁻ ¹cm⁻¹ auf.

In einer ersten bevorzugten Ausführungsform der Erfindung stellt der an Avidin oder/und Streptavidin bindefähige Ligand Biotin oder ein Biotinderivat, insbesondere Iminobiotin, Diaminobiotin oder Desthiobiotin, dar.

Vorzugsweise bedeutet R in der Verbindung (I) Wasserstoff. B bedeutet bei Semicarbaziden vorzugsweise NH und A bedeutet bei Hybraziden und Semicarbaziden vorzugsweise O.

In einer weiteren bevorzugten Ausführungsform der vorliegenden Erfindung, stellt der an Avidin oder/und Streptavidin bindefähige Ligand selbst einen Chromophor dar. In diesem Fall kann die Verbindung X - Y eine Struktur der Formel (II) aufweisen: 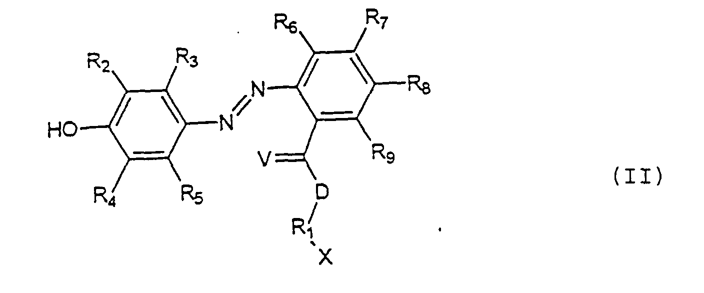 worin X wie oben definiert ist, V und D jeweils unabhängig NH, O oder S darstellen, R₁ eine gegebenenfalls Heteroatome enthaltende Alkylen-, Alkenylen- oder Alkinylengruppe mit einer Länge von 2 bis 20 Atomen darstellt und R₂ bis R₉ jeweils unabhängig Wasserstoff oder eine Alkylgruppe mit 1 bis 4 Kohlenstoffatomen darstellen. Bevorzugt stellen die Rest R₂ bis R₉ Wasserstoff dar. V ist vorzugsweise Sauerstoff. Weiterhin ist es bevorzugt, daß B eine NH-Gruppe und R₁ eine Alkylengruppe mit 2 bis 6 C-Atomen darstellt. In einer besonders bevorzugten Ausführungsform der Verbindung der Formel (II) ist der Rest Y ein 2-[2'-(4"-Hydroxybenzolazo)-benzoesäureamido]-ethyl-Rest. Am meisten bevorzugt stellt X-Y 2-[2'-(4"-Hydroxybenzolazo)benzoesäureamido]ethylsemicarbazid dar.

Verbindungen mit einer Struktur der Formel (II) können aus Verbindungen mit einer Struktur der Formel (III) 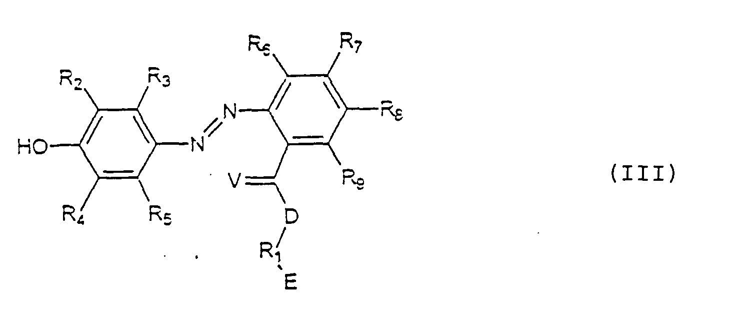 hergestellt werden, worin E eine Gruppe ausgewählt aus -OH, -SH, -NH₂ oder -COOH darstellt. Vorzugsweise bedeutet E -NH₂ oder -COOH.

In einer weiteren erfindungsgemäß bevorzugten Ausführungsform enthält in der Verbindung (I) der Rest Y eine Gruppe Y₁, die einen Chromophor umfaßt und eine Gruppe Y₂, die einen an Streptavidin oder/und Avidin bindefähigen Liganden umfaßt, sowie eine lineare gegebenenfalls Heteroatome enthaltende Verknüpfungsgruppe L mit einer Kettenlänge von 1 bis 10 Atomen, wobei entweder Y₁ oder Y₂ als Seitengruppe an L gebunden ist. Solche erfindungsgemäß bevorzugten Verbindungen weisen eine Struktur der Formel (IV) auf:

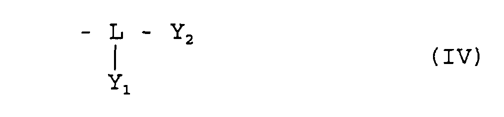

In solchen Verbindungen sind der Chromophor und die an Streptavidin oder/und Avidin bindefähige Gruppe durch eine Verknüpfungsgruppe miteinander verbunden.

Y kann einerseits die Gruppe 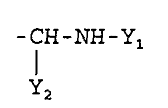 bedeuten, worin Y₁ einen Rest darstellt, der einen Chromophor enthält und Y₂ einen Rest darstellt, der einen an Streptavidin oder/und Avidin bindefähigen Liganden enthält. Der Chromophor und der bindefähige Ligand sind in dieser Ausführungsform über eine CH-NH-Gruppe miteinander verknüpft.

Weiterhin kann Y die Gruppe 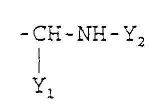 bedeuten, worin Y₁ und Y₂ wie oben definiert sind.

Grundsätzlich kann Y₁ einen beliebigen Chromophor enthalten. Bevorzugt stellt Y₁ eine Gruppe CHR-(CH₂)ₙ-Q dar, worin n = 0 bis 20, R Wasserstoff oder eine Alkylgruppe ist und Q einen aromatischen oder heteroaromatischen Rest, vorzugsweise einen Naphthylrest, darstellt.

Y₂ kann einen beliebigen Rest darstellen, der einen an Streptavidin oder/und Avidin bindefähigen Liganden enthält. Bevorzugt ist Y2 ausgewählt aus den Gruppen - ((CH₂)ₙV_{q})-K und -C(O)-(CH₂)ₙV)_{q}-K worin V NH, O oder S darstellt, K Biotin oder ein Biotinderivat ist, , n = 0 - 20, q = 0 oder 1 ist. Vorzugsweise ist Y₂ eine -((CH₂)ₙV)_{q}-K-Gruppe, worin n = 1 bis 7, q = 0 oder 1, V NH und K Biotin ist.

In Verbindungen der Formel (IV), die eine Verknüpfungsgruppe zwischen dem Chromophor und der an Streptavidin oder/und Avidin bindefähigen Gruppe enthalten, stellt X bevorzugt die Gruppe - C(A)-NR-NH₂ dar, worin A und R wie oben definiert sind. Besonders bevorzugt ist die Verbindung X-Y Biotinyl-L-3-(2-Naphthyl)alaninhydrazid. Der an Avidin oder/und Streptavidin bindefähige Biotinylrest kann auch durch ein Biotinderivat, insbesondere Iminobiotin, Diaminobiotin oder Desthiobiotin, ersetzt werden.

Die Erfindung umfaßt weiterhin die Verwendung der beschriebenen Verbindungen als Reagenz zur Derivatisierung einer eine Aldehyd-, Keton-, Semiketal- oder Semiacetal-Gruppe enthaltenden Substanz, z.B. von Sacchariden.

Ein weiterer Gegenstand der Erfindung ist ein Konjugat gebildet aus einer eine Aldehyd-, Keton-, Semiketal- oder Semiacetal-Gruppe enthaltenden Verbindung und einer Verbindung der Formel
(I). Die Verbindungen (I) reagieren selektiv mit diesen funktionellen Gruppen und bilden dabei Konjugate, die eine Bindefähigkeit an Avidin oder/und Streptavidin aufweisen und einen Chromophor enthalten. Ein derartiges Konjugat kann eine Struktur der Formel (V) aufweisen:

   Z' - X' - Y (V)

   worin Z'-X' das Reaktionsprodukt einer Aldehyd-, Keton-, Semiketal- oder Semiacetal-Funktion enthaltenden Verbindung mit der Gruppe X ist und X und Y die zuvor genannten Bedeutungen besitzen.

Insbesondere eignen sich die erfindungsgemäßen Verbindungen X-Y zur Bildung eines Konjugats mit einem Mono-, Di- oder Oligosaccharid. Es wurde überraschenderweise gefunden, daß die erfindungsgemäßen Verbindungen selektiv mit dem reduzierenden Ende des Zuckers reagieren, wobei dessen Fähigkeit zur Bildung einer geschlossenen Ringstruktur vorzugsweise erhalten bleibt. So können Saccharid-Derivate von Verbindungen der Formel (II) zu mehr als 90% in Form einer geschlossenen Ringstruktur vorliegen.

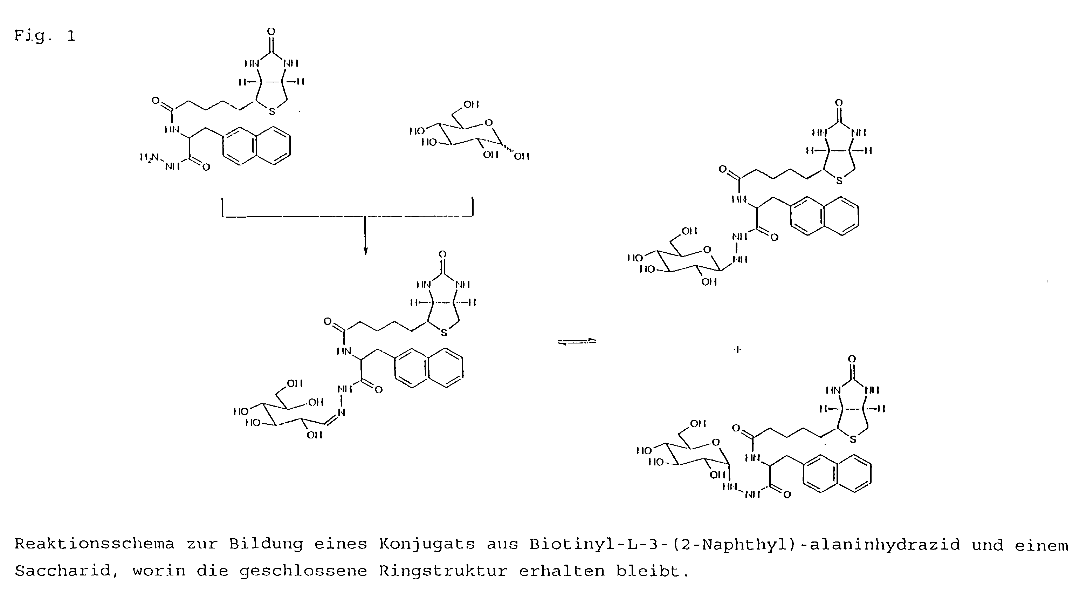

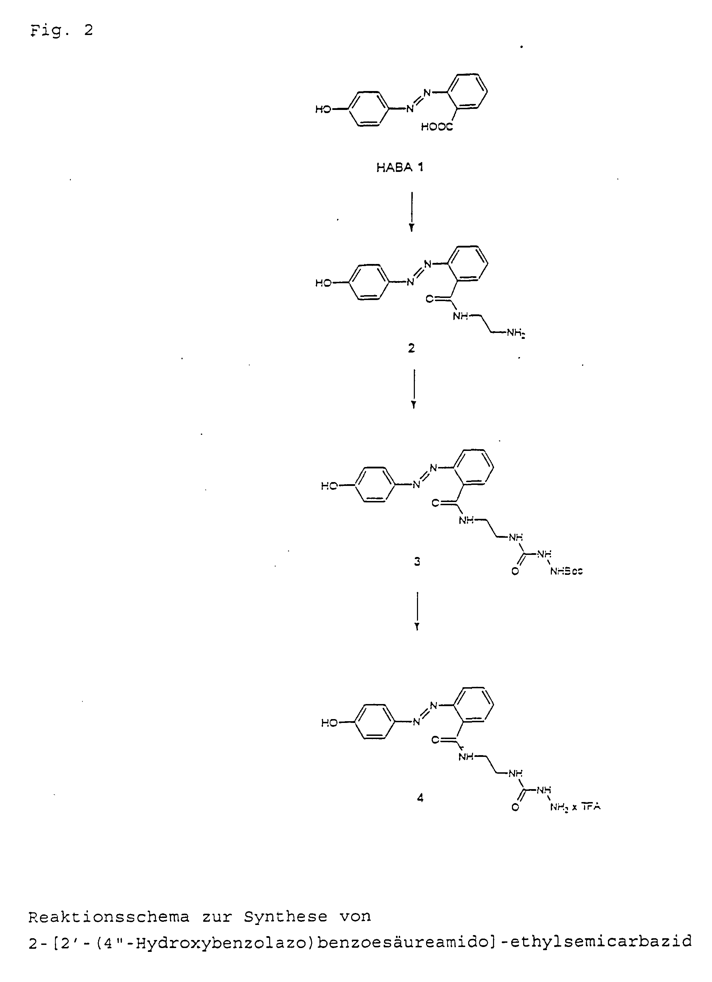

Ein Reaktionsschema zur Bildung eines erfindungsgemäßen Konjugats ist in Figur 1 veranschaulicht. Figur 2 zeigt ein weiteres Reaktionsschema, in dem die Synthese einer erfindungsgemäßen Verbindung dargestellt ist.

Besonders bevorzugt sind Konjugate aus Verbindungen (I), bei denen der bindefähige Ligand selbst ein Chromophor ist, z.B. Verbindungen (II). Weiterhin besonders bevorzugt sind Konjugate aus Verbindungen (I), bei denen Y die Struktur (IV) aufweist.

Ein weiterer Gegenstand der Erfindung ist ein Verfahren zur Herstellung eines oben beschriebenen Konjugats, worin ein Aldehyd, Keton, Semiketal oder Semiacetal mit einer erfindungsgemäßen Verbindung (I) unter Bedingungen umgesetzt wird, bei denen die Gruppe X mit der Aldehyd-, Keton-, Semiacetal- oder Semiketalgruppe reagiert. Als Aldehyd, Keton, Semiketal oder Semiacetal wird insbesondere ein Mono-, Di- oder Oligosaccharid eingesetzt.

Weiterhin umfaßt die Erfindung Komplexe, die aus einem solchen Konjugat und Avidin oder/und Streptavidin gebildet sind. Bevorzugt enthält ein solcher Komplex 1 bis 4 Moläquivalente des Konjugats pro Moläquivalent Avidin oder Streptavidin. Besonders bevorzugt liegt das Avidin oder Streptavidin immobilisiert an einer Festphase vor.

Diese Komplexe können als Immunogen zur Herstellung von Anti-Glycan-Antikörpern eingesetzt werden. Hierzu werden die Komplexe vorzugsweise mit einem Adjuvans, z.B. komplettem Freund'schen Adjuvans, versetzt und mehrmals in zeitlichen Intervallen einem Versuchstier, z.B. einer Maus verabreicht. Aus diesem Versuchstier können dann auf bekannte Weise Antikörper gegen die Zuckerkomponente des Komplexes gewonnen werden. Zur genauen Versuchsdurchführung wird auf Beispiel 5 von WO 94/28008 verwiesen.

Mittels der erfindungsgemäßen Verbindungen, Konjugate und Komplexe ist es möglich, Kohlehydrate selektiv zu fraktionieren und sensitiv zu detektieren, wodurch Kohlehydrate nachgewiesen und isoliert sowie Wechselwirkungen von Kohlehydraten mit Rezeptoren untersucht werden können. Die Erfindung umfaßt deshalb weiterhin ein Verfahren zum Nachweis oder zur Isolierung von Kohlehydraten umfassend die Schritte:
(a) Inkontaktbringen einer erfindungsgemäßen Verbindung (I) mit einer ein nachzuweisendes oder/und zu isolierendes Kohlehydrat enthaltenden Probe,
(b) Bildung eines Konjugats aus nachzuweisendem Kohlehydrat und der Verbindung (I) und
(c) Nachweis oder/und Isolierung des Konjugats.

Hierzu können die Verbindungen (I) einerseits (i) zu den Konjugaten umgesetzt und (ii) danach durch Inkubation mit Steptavidin oder/und Avidin komplexiert werden. Andererseits können die Verbindungen (I) auch (i) zuerst komplexiert und (ii) danach zu den Konjugaten umgesetzt werden.

Erfindungsgemäß bevorzugt wird an eine Festphase wie z.B. eine Mikrotiterplatte, eine Mikroreaktionsgefäß, eine Membran, z.B. eine Nitrocellulose - oder PVDF-Membran, oder gegebenenfalls magnetische Mikrobeads, oder ein Makromolekül gebundenes Avidin oder/und Streptavidin verwendet, um das Konjugat zu immobilisieren. Der Nachweis bzw. die Isolierung der Kohlehydrate erfolgt bevorzugt durch Bindung eines Glycanrezeptors. Glycanrezeptoren sind Substanzen, die spezifische Wechselwirkungen mit Kohlehydraten definierter Struktur eingehen. Beispiele für Glycanrezeptoren, die zum Nachweis oder/und zur Isolierung spezifischer Kohlehydratstrukturen verwendet werden können, sind Selectine, Lectine und Anti-Glycan-Antikörper. Lectine und Selectine binden sehr spezifisch an bestimmte Kohlehydrat-Strukturen. Das Lectin MAA bindet beispielsweise spezifisch an α(2-3) gebundene Sialinsäuren, was den Nachweis dieser Gruppe ermöglicht. Lectine sind kommerziell erhältlich, z.B. von Boehringer Mannheim, siehe Katalog Biochemicals.

Zum Nachweis von Kohlehydraten wird bevorzugt ein Glycanrezeptor eingesetzt, der direkt oder indirekt markiert ist. Unter direkter Markierung ist dabei zu verstehen, daß der Glycanrezeptor selbst eine Markierungsgruppe trägt, während unter indirekter Markierung zu verstehen ist, daß ein weiteres Reagenz, das mit dem Glycanrezeptor bindefähig ist, die Markierung trägt.

Die Markierung kann eine beliebige Markierung sein, insbesondere kann die Markierung radioaktiv, über ein Hapten, ein Enzym, einen Metallkomplex, einen Lumineszenz- oder Fluoreszenz-Marker erfolgen. Weiterhin kann erfindungsgemäß bevorzugt die Bestimmung des Glycanrezeptors über einen gegebenenfalls markierten, spezifischen Antikörper erfolgen. Lectine können z.B. Digoxigenin markiert verwendet werden, wobei ein Nachweis mit markiertem Anti-Digoxigenin Antikörper erfolgen kann.

Andererseits ermöglichen Konjugate der erfindungsgemäßen Verbindungen mit Sacchariden auch einen spezifischen Nachweis von Glycanrezeptoren, d.h. Glycan-bindenden Proteinen, Lectinen etc. Ein weiterer Gegenstand der vorliegenden Erfindung ist somit ein Verfahren zum Nachweis oder/und zur Isolierung von Glycanrezeptoren, umfassend die Schritte:
(a) Bildung von Konjugaten aus einem oder mehreren ausgewählten und somit bekannten Kohlehydraten und einer erfindungsgemäßen Verbindung (I)
(b) Inkontaktbringen des Konjugats mit einer einen nachzuweisenden oder/und zu isolierenden Glycanrezeptor enthaltenden Probe und
(c) Nachweis oder/und Isolierung des Glycanrezeptors.

Hierzu können einerseits die Konjugate (i) durch Inkubation mit Streptavidin oder/und Avidin in einen Komplex überführt und (ii) danach mit der Probe in Kontakt gebracht werden. Andererseits können auch die Konjugate (i) mit der Probe in Kontakt gebracht und (ii) danach durch Inkubation mit Streptavidin oder/und Avidin in einen Komplex überführt werden. Vorzugsweise wird das Streptavidin oder/und Avidin an eine Festphase gebunden, so daß der Glycanrezeptor über seine Bindung an die Festphase aus der Probe abgetrennt und in isolierter Form nachgewiesen werden kann. Spezifische Beispiele für nachzuweisende Glycanrezeptoren werden in einem Übersichtsartikel von Varki (Glycobiol. 3 (1993), 97 - 130) genannt, wie etwa Lectine, Selectine oder zelluläre Kohlehydrat-enthaltende Oberflächenstrukturen, z.B. E-Selectin, P-Selectin, L-Selectin, CD44, CD22β oder Makrophagen-Gal/Nac-Rezeptor. Weiterhin können durch das Verfahren auch noch unbekannte Glycanrezeptoren identifiziert und isoliert werden.

Zum Nachweis von Glycanrezeptoren können erfindungsgemäße Konjugate und Komplexe in markierter Form eingesetzt werden.

Auch der nachzuweisende oder/und zu isolierende Glycanrezeptor kann gegebenenfalls markiert sein, z.B. durch eine in vivo Markierung mit radioaktiven Isotopen wie ³⁵S oder ¹⁴C. Andererseits kann die Markierung auch vor oder nach Bindung des Rezeptors an das Konjugat durch chemische Kopplung, z.B. mit Aktivestern oder SH-Reagenzien eingeführt werden.

Der Nachweis bzw. die Isolierung des Glycanrezeptors erfolgt vorzugsweise über seine Bindung an eine Festphase, wodurch er von den übrigen Bestandteilen der Probe abgetrennt werden kann. Nach dieser Abtrennung kann der Glycanrezeptor, sofern gewünscht, wieder von der Festphase abgelöst werden, z.B. durch Verdrängung oder Zugabe eines Denaturierungsreagenz. Der abgelöste Glycanrezeptor, der gegebenenfalls in markierter Form vorliegt, kann anschließend durch geeignete Methoden weiter charakterisiert werden, z.B. durch Gelelektropherese wie etwa SDS-PAGE oder durch einen immunologischen Test wie etwa einen ELISA.

Weiterhin umfaßt die Erfindung einen Reagenzienkit zum Nachweis oder/und zur Isolierung von Kohlehydraten umfassend
(a) eine Verbindung (I) und
(b) eine mit Streptavidin oder/und Avidin beschichtete Festphase.

Als Festphase können z.B. eine Mikrotiterplatte, ein Mikroreaktionsgefäß, eine Membran oder gegebenenfalls magnetische Mikrobeads verwendet werden. Zudem kann der Reagenzienkit weitere Reagenzien enthalten, die zur Bestimmung von Kohlehydraten verwendet werden können. Dazu gehören z.B. Glycanrezeptoren, wie etwa Digoxigenin-markierte Lectine, und Kohlehydrat-spaltende Enzyme.

Noch ein weiterer Gegenstand der vorliegenden Erfindung ist ein Reagenzienkit zum Nachweis oder/und zur Isolierung von Glycanrezeptoren, umfassend:
(a) eine erfindungsgemäße Verbindung, die gegebenenfalls in Form eines Konjugats mit einem für den nachzuweisenden oder/und zu isolierenden Glycanrezeptor spezifischen Saccharid vorliegen kann und
(b) eine mit Streptavidin oder/und Avidin beschichtete Festphase.

Die nachfolgend angeführten Beispiele und beigefügten Figuren veranschaulichen die Erfindung näher, wobei
- Figur 1: ein Reaktionsschema zur Bildung eines Konjugats aus Biotinyl-L-3-(2-Naphthyl)-alaninhydrazid mit einem Saccharid darstellt;
- Figur 2: ein Reaktionsschema zur Synthese der Verbindung 2-[2'-(4"-Hydroxybenzolazo)-benzoesäureamido]ethylsemicarbazid zeigt (Beispiel 1) ;
- Figur 3: das Chromatogramm einer Umsetzung von derivatisiertem Dextranhydrolysat mit HABA-Semicarbazid (15 µg) auf einer Mikrosorb-NH2-Säule zeigt (Beispiel 3);
- Figur 4: das RP18 Chromatogramm der Umsetzung von je 0,5 nmol biantennärem Decasaccharid (2) und Undecasaccharid (1) mit HABA-Semicarbazid auf einer ODS-Hypersilsäule zeigt (Beispiel 3);
- Figur 5a: die Spaltstellen der Enzyme N-Glycosidase-F und Endo F₂ an dem Saccharidanteil von humanem Transferrin zeigt (Beispiel 3a) ;
- Figur 5b: das RP18 Chromatogramm des mittels Endo F2 (1) bzw. N-Glycosidase F(2) abgespaltenen Saccharidanteils von humanem Transferrin nach Umsetzung mit HABA-Semicarbazid auf einer ODS-Hypersilsäule zeigt (Beispiel 3a) ;
- Figur 6: das Chromatogramm des mittels N-Glycosidase F abgespaltenen Saccharidanteils von Fetuin nach Derivatisierung mit HABA-Semicarbazid vor (unten) und nach (oben) Verdau der Zuckerderivate mit Neuraminidase auf einer Anionenaustauschersäule zeigt (Beispiel 3b) ;
- Figur 7: die RP18 Chromatogramme einer sequentiellen Abspaltung von Zuckerresten von einem bianntenären Undecasaccharid mit mehreren Exoglycosidasen darstellt (Beispiel 4);
- Figur 8a: die Spaltstellen der Enzyme Neuraminidase, Galactosidase, N-Acetylglucosaminidase und Mannosidase an einem biantennären Undecasaccharid zeigt (Beispiel 4);
- Figur 8b: die Detektion von Saccharidderivaten aus Fragmenten des in Fig. 8a gezeigten Saccharids, die durch Verdau mit verschiedenen Enzymen erzeugt wurden, mit Digoxigenin-markierten Lectinen (SNA, DSA und GNA) zeigt (Beispiel 4);
- Figur 9: die Detektion steigender Mengen an Fetuinzuckerderivaten mit den Digoxigenin-markierten Lectinen SNA und MAA zeigt (Beispiel 4);
- Figur 10: den Einfluß steigender Mengen an N-Acetylneuraminosyl-2,3-D-Lactose-Derivat bei der Detektion von Fetuinzuckerderivaten mit Digoxigenin-markierten Lectinen SNA und MAA zeigt (Beispiel 4);
- Figur 11: die Detektion von Fetuinzuckerderivaten (10pmol) vor (1) und nach (2) Verdau mit NDV-Sialidase mit den Digoxigenin-markierten Lectinen MAA und SNA zeigt (Beispiel 4) und
- Figur 12: den Nachweis unterschiedlicher Mengen von 3'-Sialyl Lewis X (Balken 1 und 2) und 3'-Sulfatyl Lewis X (Balken 3 und 4) mit einem E-Selectin Fusionsprotein darstellt (Beispiel 4).

Die nachfolgend aufgeführten Beispiele erläutern die Erfindung weiter:

### Beispiel 1:

### Darstellung von 2-[2'-(4"-Hydroxybenzolazo)-benzoesäureamido]ethylsemicarbazid 4

Die Synthese von 2-[2'-(4''-Hydroxybenzolazo)benzoesäureamido]-ethylsemicarbazid läuft nach dem in Fig. 2 dargestellten Reaktionsschema ab.

### Darstellung von 2-[2'-(4"-Hydroxybenzolazo)-benzoesäureamido]ethylamin 2

Zu einer Lösung von 6 g (25 mmol) 2-(4'-Hydroxybenzolazo)benzoesäure **1** (HABA) (Aldrich) und 3 g (25 mmol) NHS (97%, Fluka) in 50 ml trockenem Tetrahydrofuran (THF) wird unter Rühren eine Lösung von 10.3 g (50 mmol) N, N'Dicyclohexylcarbodiimid (DCC) (Fluka) in 50 ml THF zugetropft und noch 24 h bei RT weitergerührt. Anschließend wird vom entstandenen Niederschlag abgesaugt, das Filtrat zur Trockne eingeengt und das erhaltene Rohprodukt über Kieselgel (AcOEt:MeOH = 6:1) filtriert. Nach Entfernung des Lösungsmittels wird der erhaltene Rückstand in 50 ml THF/MeOH (1:1) gelöst und bei RT zu einer Lösung von 5 ml Ethylendiamin (75 mmol) (99%, Aldrich) getropft. Die Reaktionslösung wird 0.5 h weitergerührt, dann bis zur Trockne eingeengt und das Produkt durch Chromatographie (Kieselgel, MeOH) gereinigt. Man erhält 1,5 g (20% über 2 Stufen) von Verbindung **2**, als orangen Feststoff.

### Darstellung von 3.

Zu einer Lösung von 0.8 g (5 mmol) CDI (Aldrich) in 10 ml wasserfreiem DMF wird nacheinander unter Rühren 0.66 g (5 mmol) t-Butylcarbazol (99%, Aldrich) und eine Lösung von 1.5 g (5 mmol) **2** in 20 ml wasserfreiem DMF gegeben. Man rührt noch 1 h bei RT nach, gießt dann das Reaktionsgemisch in 200 ml Wasser und extrahiert mit 2 x 200 ml Essigsäureethylester. Die vereinigten organischen Phasen werden nacheinander mit 200 ml 1 N HCl, 200 ml Wasser, 200 ml 0,1 M Phosphat-Puffer, pH 7.0 und 200 ml Wasser gewaschen und getrocknet (MgSO₄). Schließlich filtriert man ab und befreit das Filtrat vom Lösungsmittel. Das Rohprodukt wird zuerst filtriert (Sepharose LH 20, MeOH) und schließlich durch Säulenchromatographie (Kieselgel, Toluol:Aceton:MeOH = 1:1:0,1) gereinigt. Man erhält 950 mg (40 %) **3** als orangen Feststoff.
¹H NMR (100 MHz, CD₃OD): δ = 1.44 (s, 9 H, Boc-Protonen); 3.30-3.61 (m, 4 H, N-CH₂-CH₂-N) ; 6.95 und 7.83 (4 H, AA'BB'-System, H-C(2"), H-C(3"), H-C(5") und H-C(6")); 7.48 - 7.91 (m, 4 H, Protonen an C(3') bis C(6')).

### Darstellung von 4.

Zu einer Lösung von 218 mg (0.5 mmol) **3** in 2 ml CH₂Cl₂ werden 2 ml TFA zugesetzt und 30 min bei 40°C gerührt. Anschließend zieht man das Lösungsmittel im Vakuum ab, löst den Rückstand in wenig MeOH, gibt 10 ml Wasser hinzu und lyophilisiert. Man erhält 205 mg (93 %) **4** als orangen Feststoff. Das Produkt kann direkt zur Markierung von Aldehyden und Ketonen eingesetzt werden.
MS [M + H] 343.1.
¹H NMR (500 MHz, D₆-DMSO): δ = 3.26 (ψq, 2 H, H₂-C(1)); 3.37 (ψq, 2 H, H₂-C(2)); 6.96 und 7.77 (AA'BB'-System, 4 H, H-C(2"), H-C(3"), H-C(5") und H-C(6")); 7.23 (t, 1 H, C(1)-NH-C(O)-N); 7.51 - 7.59 und 7.64 - 7.70 (2 m, 4 H, Protonen an C(3') bis C(6')); 8.48 (t, 1 H, Ar-C(O)-HN-C(2)); 8.83 (br s, 1 H, C(O)-NH-N); 9.75 (br s, 2 H, N-NH₂); 10.5 (s, 1 H, phenolisches OH)).

### Beispiel 2:

### Derivatisierung von Sacchariden mit 2-[2'-(4"-Hydroxybenzolazo)-benzoesäureamido]ethylsemicarbazid.

### - Umsetzung von Glucose:

Zu einer Lösung von 11.4 mg (0.06 mmol) Glucose (Merck) in 1 ml H₂O wird eine Lösung von 20.5 mg (0.06 mmol) des Markers in 5 ml MeOH:AcOH (10:1; v:v) zugesetzt und 18 h bei 60°C gerührt. Anschließend wird das Lösungsmittel im Vakuum abgezogen, der Rückstand in wenig MeOH aufgelöst, 10 ml Wasser hinzugegeben und der Ansatz anschließend lyophilisiert. Das Rohprodukt wird durch Säulenchromatographie (Sepharose LH 20, MeOH bzw. RP18, 0.1 % TFA in 17 % CH₃CN) gereinigt. Man erhält 17 mg (56 %) des Glucosekonjugats als orangen Feststoff.
UVₘₐₓ: 360 nm (∈₃₆₀ = 17000 1 mol⁻¹cm⁻¹).
MS [M + H]: 505.2
¹³C NMR (500 MHz, D₆-DMSO + D₂O) : δ = 91.2 (C-1), 70.5 (C-2), 77.2 (C-3), 71.4 (C-4), 77.8 (C-5), 62.0 (C-6); 39.2 (H₂-C(1)) ; 39.9 (H₂-C(2)) ; 115 - 150 (C-Atome des Aromaten); 160.3 (Ar-C(O)-HN-C(2)) ; 168.1 (NH-C(O)-NH).
¹H NMR (500 MHz, D₆-DMSO + D₂O) : δ = 3.7 (1 H; H-C(1)), 3.0 (1 H; H-C(2)), 3.2 (1 H; H-C(3)), 3.0 (1 H; H-C(4)), 3.1 (1 H; H-C(5)), 3.7 (2 H; H₂-C(6)) ; 3.3 (2 H, H₂-C(1')) ; 3.5 (2 H, H₂-C(2')) ; 6.6 und 7.6 (AA'BB'-System, 4 H, H-C(2"), H-C(3"), H-C(5") und H-C(6")) ; 7.5 - 7.6 und 7.3 (4 H, Protonen an C(3') bis C (6')).
Aus den NMR-Daten kann geschlossen werden, daß das Glucosederivat eine geschlossene Ringstruktur (β-Konformation) besitzt. Dies konnte auch durch eine Strukturberechnung bestätigt werden.

### - Umsetzung von Mono- und Oligosacchariden:

### a) Umsetzungsraten:

Für einen Vergleich der Umsetzungsraten unterschiedlicher Saccharide für analytische Zwecke werden in verschließbaren Eppendorfcaps in 100 µl-Ansätzen je 1 nmol von Glucose, N-Acetyl-neuraminosyl-2,3-D-Lactose (Boehringer Mannheim), biantennärem Undecasaccharid (BioCarb) und einem triantennären sialyliertem Oligosaccharid (Oxford Glyco Systems) mit dem fünffachen molaren Überschuß an Marker **4** in MeOH 12 h bei 60°C umgesetzt. Die Umsetzungsrate wird mittels RP18 Chromatographie (ODS-Hypersil 250 x 4.6 mm der Firma Bischoff; Eluent A: 0.1 % TFA in H₂O; Eluent B: 0.1 % TFA in CH₃CN; Gradient 0 - 40 % in 30 min) und über DC (Laufmittel:MeOH:H₂O:Butan-2-on = 6:2:2; v:v:v; Detektion der Zucker mittels 20 % H₂SO₄ in EtOH) bestimmt.

Auf der DC konnte nach der Derivatisierung kein freier Zucker mehr nachgewiesen werden, was eine Umsetzungsrate > 95 % anzeigt. Bei der HPLC-Analyse waren die Flächen der Umsatzsignale für alle Saccharide vergleichbar, was eine gleiche Umsetzungsrate für die verschiedenen Saccharide anzeigt. Die Nachweisgrenze der Saccharidderivate mittels UV-Detektion liegt bei ca. 100 pmol. Der Marker eluiert bei ca. 30 % CH₃CN von der RP18-Säule und kann so leicht von den Konjugaten getrennt werden, die bei 10 - 20 % CH₃CN eluieren.

### b) Stabilitäten:

Die Saccharidkonjugate zeigen in Lösung bei 25°C (40 h) bzw. nach Lyophylisation keine Zersetzungserscheinungen (nach HPLC-Analyse < 5 %). Der Marker **4** selber zeigt nach einer Woche in MeOH bei 25°C noch keine Anzeichen einer Zersetzung.

### c) Abtrennung des Marker-Überschusses:

Die Abtrennung des Marker-Überschusses nach Umsetzung mit den Sacchariden erfolgt durch Extraktion mit Essigsäureethylester. Dazu werden zu den 100 µl Ansätzen 100 µl H₂O bidest. und 800 µl Essigsäureethylester gegeben; nach Durchmischen und Zentrifugation (1 min bei 10000 g) wird die obere Essigesterphase enffernt und die Extraktion mit der unteren wässrigen Phase noch zweimal wiederholt. Dabei können 90 % des Markers abgetrennt werden. Der Verlust an Zuckerkonjugat, getestet mit einem Glucosekonjugat, liegt unter der Nachweisgrenze von 5 %.

### Beispiel 3:

### Auftrennung von derivatisierten Sacchariden auf HPLC-Säulen.

### - Analyse eines Dextranhydrolysats:

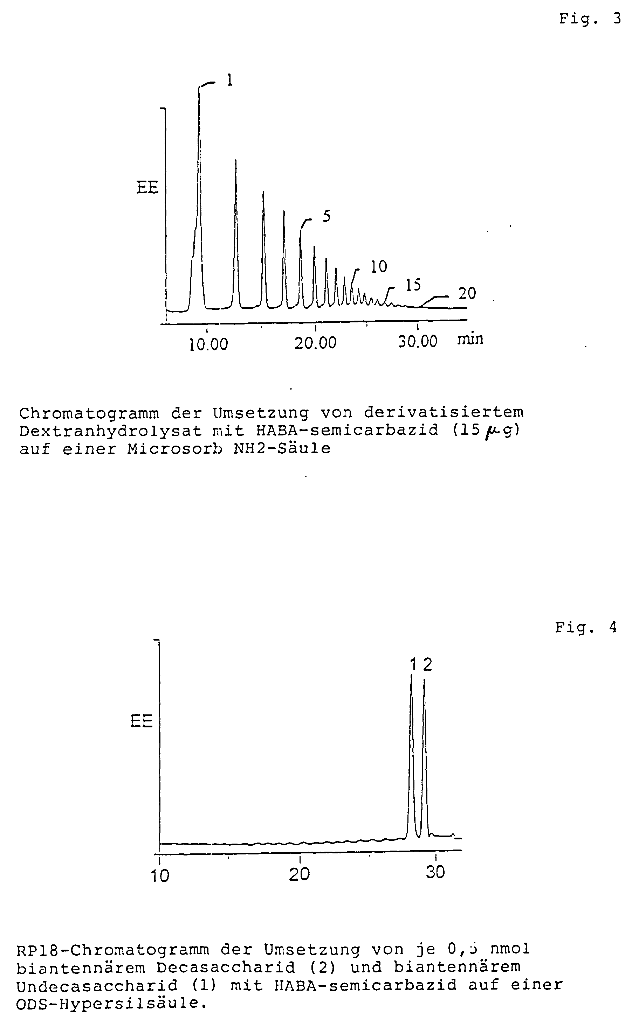

Ein Dextranhydrolysat (Oxford Glyco Systems) wird mit einem Marker konjugiert und analysiert. Mit einer Carbohydrate CHO C18 Säule (220 x 2.1 mm; Perkin Elmer) und einem linearen Gradienten von 25 - 75 % in 50 min (Eluent A: 0.1 M NH₄OAc pH 5.5 mit 10 % CH₃CN; Eluent B: 0.1 M NH₄OAc pH 5.5 mit 25 % CH₃CN; Flußrate 0.2 ml/min) wird bei einer Auftragungsmenge von 5 µg des Dextranhydrolysatkonjugats eine Aufspaltung vom Glucosemonomer bis zum siebzehnmer Oligosaccharid erhalten. Bei Analyse des gleichen mit Marker 4 konjugierten Dextranhydrolysats mit einer Microsorb-NH2 Säule (250 x 4.5 mm; Rainin) unter den unten angegebenen Eluationsbedingungen kann bei 15 µg Auftragungsmenge eine Auftrennung bis zum 20-mer Oligosaccharid erhalten werden (Fig. 3).
- Eluent A:: 10 mM NH₄OAc pH 3.0
- Eluent B:: 10 mM NH₄OAc pH 3.0 in H₂O/CH₃CN 5:95
- Flußrate:: 0.8 ml/min

### Gradient:

| Zeit (min) | % Eluent A | % Eluent B |
|---|---|---|
| 0 | 5 | 95 |
| 20 | 40 | 60 |
| 60 | 60 | 40 |

### - Auftrennung von Monosacchariden:

Als Monosaccharidgemisch werden je etwa 1 nmol derivatisierter Glucose, Fucose, Xylose und Fructose der Firma Merck auf einer ODS-Hypersilsäule analysiert. Der lineare Gradient beträgt 10 - 30 % in 40 min mit Eluent A: 0.2 % Tetramethylethylendiamin in H₂O/H₃PO₄ pH 4.5 und Eluent B: CH₃CN bei einer Flußrate von 1 ml/min [Anumula K. R., (1994), Anal. Biochem. 220; 275]. Die Monosaccharide können voneinander getrennt werden.

### - Analyse von acetylierten Zuckern:

Je etwa 1 nmol N-Acetyl-neuraminosyl-2,3-D-Lactose und N-Acetyl-Neuraminsäure (Sigma), je 0.5 nmol N,N'-Diacetylchitobiose (Sigma) und N,N',N'',N'''-Tetraacetylchitotetraose (Sigma) werden mit Marker **4** derivatisiert und zusammen mit 1 nmol derivatisierter Glucose als Vergleichssubstanz auf einer ODS-Hypersilsäule analysiert. Der Gradient beträgt 10 - 25 % in 40 min mit Eluent A: 0.1 % TFA in H₂O und Eluent B: 0.1 % TFA in CH₃CN bei einer Flußrate von 1 ml/min. Auch hier kann eine gute Auftrennung des Gemisches erzielt werden.

### - Analyse von Oligomaltosen:

Bei der Analyse von derivatisierten Oligosacchariden werden etwa je 1 nmol derivatisierte Maltose, Maltotetraose, Maltopentaose und Maltoheptaose von der Firma Merck und als Vergleich 1 nmol derivatisierte Glucose auf eine ODS-Hypersilsäule aufgetragen. Der Gradient beträgt 10 - 30 % in 40 min mit Eluent A: 0,1 % TFA in H₂O und Eluent B: 0.1 % TFA in CH₃CN bei einer Flußrate von 1 ml/min. Die Saccharide werden unter diesen Bedingungen vollständig aufgetrennt

### - Auftrennung von biantennären Oligosacchariden:

Analysiert werden je 0.5 nmol von derivatisierten biantennären sialylierten Deca- und Undecasaccharid (BioCarb) auf einer ODS-Hypersilsäule. Der Gradient beträgt 0 - 30 % in 40 min mit Eluent A: 0.1 % TFA in H₂O und Eluent B: 0.1 % TFA in CH₃CN bei einer Flußrate von 1 ml/min. Die sich nur um einen Zuckerrest unterscheidenden Saccharide werden vollständig aufgetrennt (Fig. 4).

### - Analyse von Oligosacchariden von Glycoproteinen:

Für die Deglycosylierung von Glykoproteinen mit dem Enzym N-Glycosidase F (Boehringer Mannheim) wird das Glykoprotein (1 mg/ml) in 1 %iger SDS-Lösung durch Erhitzen auf 100°C denaturiert. Zu 20 µl des denaturierten Glykoproteins werden anschließend 40 µl 0.5 M Natriumphosphat pH 7.2, 10 µl Wasser, 20 µl Nonidet P40® und 10 µl N-Glycosidase F (200 U/ml) zugegeben. Die vollständige Deglycosylierung wird nach 1 h bei 37°C erreicht. In einem weiteren Ansatz wird eine Spaltung mit Endo F2 (400 U/ml; Boehringer Mannheim) in 0.5 M Natriumacetat pH 4.5 unter ansonsten gleichen Bedingungen durchgeführt. Da die Oligosaccharide bei der Spaltung mit N-Glycosidase F als Aminderivate anfallen (Hauffe D., GIT Fachz. Lab. 11 (1994), 1220 - 24), werden diese durch Ansäuern auf pH 4.5 (2 h bei 25°C) in die OH-Form überführt. Zur weiteren Umsetzung der Oligosaccharide mit dem Marker **4** werden die Proteine durch Ultrafiltration mit einem Microconkonzentrator (Microcon 10) der Firma Amicon von dem Ansatz abgetrennt.

### a) humanes Transferrin:

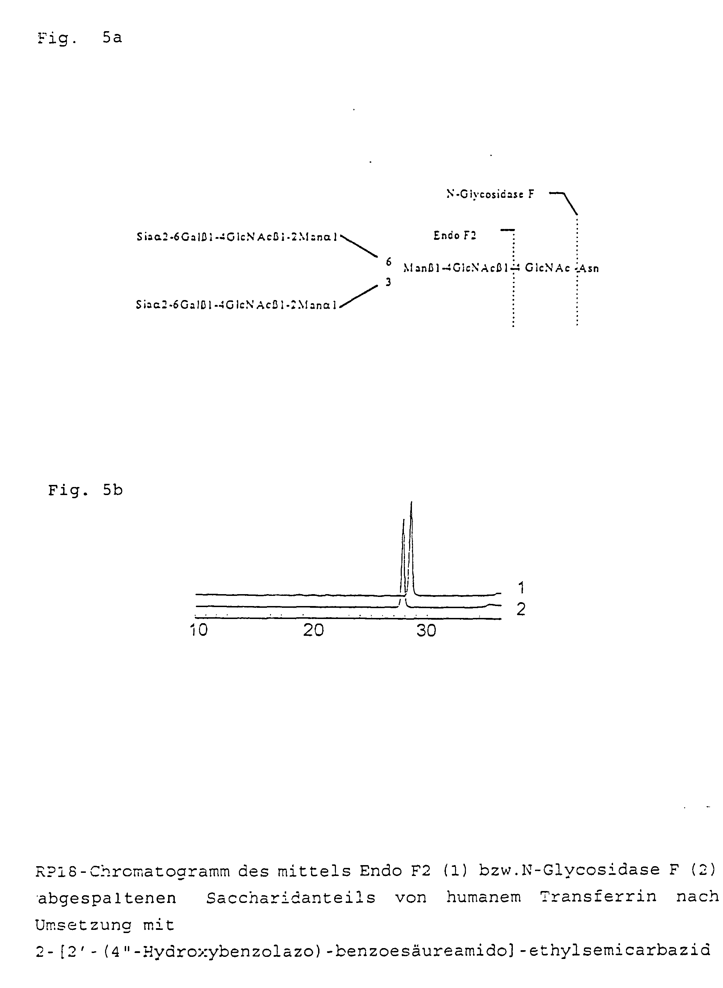

In Fig. 5a ist die in der Literatur beschriebene Struktur des humanen Transferrin-Oligosaccharids (Jamieson G. A., Jett M. & De Bernardo S. L., J. Biol. Chem. 246 (1971), 3686 - 3693) sowie die Spaltstellen der Endo F2 und N-Glycosidase F dargestellt. Die mittels Endo F2 bzw. N-Glycosidase F abgespaltenen Oligosaccharide von 50 µg humanen Transferrin (Boehringer Mannheim) ergeben nach der Derivatisierung auf der RP-HPLC je ein Signal (Fig. 5b). Die Retentionszeiten der durch die beiden Enzymen abgespaltenen Oligosaccharide auf der ODS-Hypersilsäule (Gradient 0 - 30 % in 40 min; Eluent A: 0.1 % TFA in H₂O, Eluent B: 0.1 % TFA in CH₃CN; Flußrate 1 ml/min) stimmen mit den beiden Standardsacchariden biantennäres Deca- und Undecasaccharid überein (Fig. 4).
Bei Auftragung des mittels N-Glycosidase F abgespaltenen, sialylierten Zuckerderivats auf eine Mono Q HR 5/5-Säule mit dem flüchtigen Eluenten Triethylamin/Essigsäure (Eluent A: 10 % CH₃CN mit Triethylamin auf pH 9.3 eingestellt und Eluent B: 10 % CH₃CN in 0.15 M Triethylamin mit Essigsäure auf pH 7.3 eingestellt) wird ein Signal bei etwa 20 % Eluent B erhalten.

### b) Fetuin:

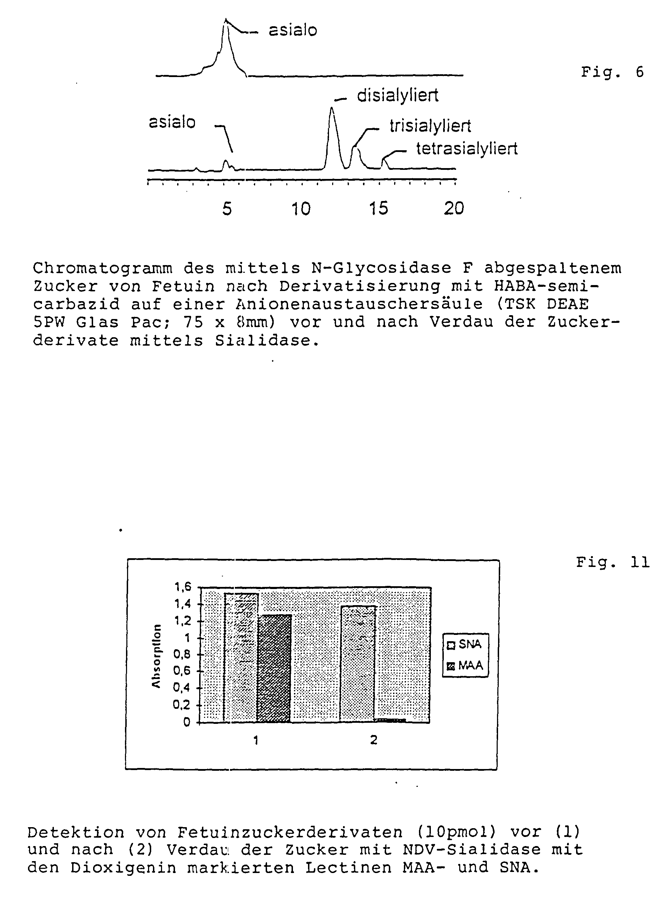

Nach Abspaltung der Zuckerreste von 60 µg Fetuin (Boehringer Mannheim) mittels N-Glycosidase F ergibt die Analyse der Oligosaccharidderivate auf einer Anionenaustauschersäule (TSK DEAE - 5PW Glas Pac; 75 x 8 mm der Firma LKB) unter den unten angeführten Eluationsbedingungen drei Signale, die von di-, tri- und tetrasialylierten Zuckern stammen (Fig. 6 unten). Die drei sialylierten Hauptsignale werden auch bei Green und Baenzinger [Green E. D. & Baenzinger J. U., Anal. Biochem. 158 (1986), 42 - 49) gefunden, allerdings in einem anderen Verteilungsmuster.
Bei Verdau der Fetuinzuckerderivate mittels Neuramidase (60 mU aus C. perfringens; Boehringer Mannheim) 2h bei 37°C kann nach Abspaltung der Sialinsäurereste keine Bindung der Oligosaccharidderivate auf der Anionenaustauschersäule mehr beobachtet werden. Es wird lediglich das asialo-Signal beobachtet (Fig. 6 oben).
- Eluent A:: MeOH
- Eluent B:: 0.1 M NaCl in MeOH
- Flußrate:: 1 ml/min

### Gradient:

| Zeit (min) | % Eluent A | % Eluent B |
|---|---|---|
| 0 | 100 | 0 |
| 15 | 60 | 40 |
| 20 | 0 | 100 |

### c) Ribonuclease B:

Die Abspaltung von High Mannose Typ Zuckerresten von 20 µg Ribonuclease B (Perkin Elmer) mittels N-Glycosidase F ergibt nach Derivatisierung der Zucker bei Analyse auf einer ODS-Hypersilsäule (Gradient 0 - 30 % in 40 min; Eluent A: 0.1 % TFA in H₂O, Eluent B: 0.1 % TFA in CH₃CN; Flußrate 1 ml/min) drei aufgelöste Signale. Die Verteilung und Intensität der Peaks entspricht den in der Literatur bekannten Beobachtungen (Kakehi K., Suzuki S., Honda S. & Lee Y. C., Anal. Biochem. 199 (1991), 256 - 268). Bei der Kontrolle mit Ansätzen von Ribonuclease B ohne Enzym wird kein Signal beobachtet.

### - Deglycosilierung von biantennärem Undecasaccharid mit Exoglycosidasen:

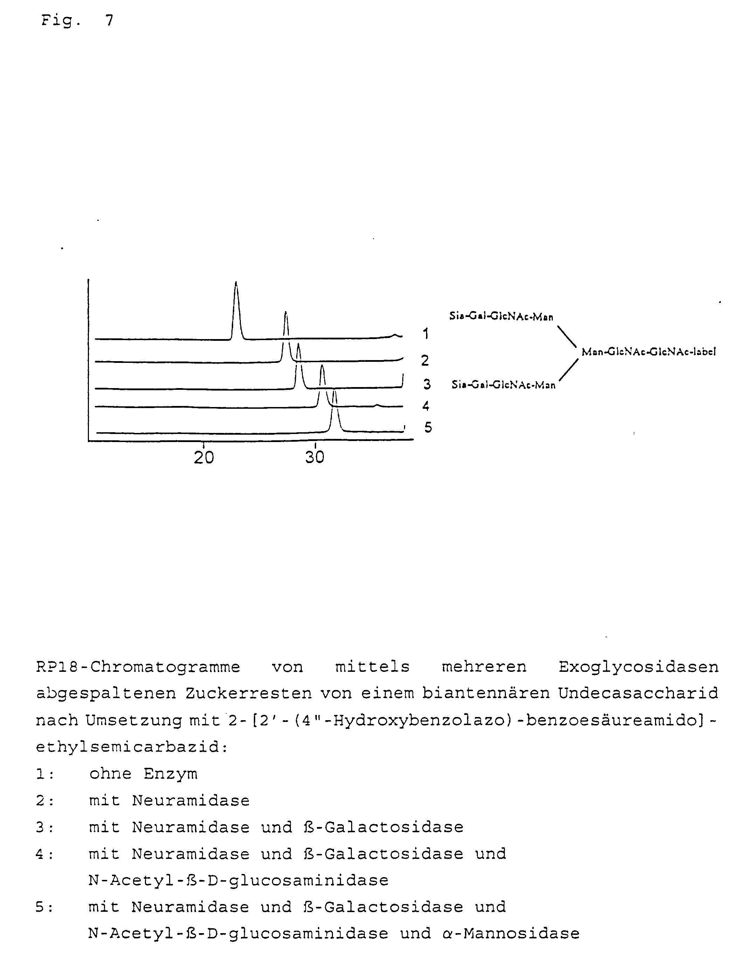

Die sequentielle Abspaltung von Zuckerresten mit Exoglycosidasen von einem derivatisierten biantennärenn Undecasaccharid mit anschließender RP-HPLC Analyse der Zuckerreste auf einer ODS- Hypersilsäule (Gradient 10 - 30 % in 40 min; Eluent A: 0.1 % TFA in HO, Eluent B: 0.1 % TFA in CH₃CN; Flußrate 1 ml/min) ergibt eine deutliche Verschiebung im Retentionsverhalten der Zucker (Fig. 7). Die Abspaltung der Sialinreste mittels Neuraminidase führt dabei erwartungsgemäß zu der größten Veränderung im Retentionsverhalten .
Für den Exoglycosidaseverdau werden je 1 nmol des derivatisierten Zuckerderivats in 100 µl 50 mM NaOAc.Puffer pH 5.5 1 h bei 37°C mit folgenden Enzymkombinationen von Boehringer Mannheim umgesetzt:
50 mU Neuramidase (C. perfringens), 8 U β-Galactosidase (E. coli); 40 mU N-Acetyl-β-D-glucosaminidase (Diplococcus pneumoniae) und 500 mU α-Mannosidase (Jack bean).

### Beispiel 4:

### Bindungsstudien mit Saccharidkonjugaten auf Streptavidin-Mikrotiterplatten.

Auf mit Streptavidin beschichteten Mikrotiterplatten (Boehringer Mannheim), mit einer Kapazität von ≥ 20 pmol Biotin pro Well (= einzelne Vertiefung der Mikrotiterplatte), können Saccharidkonjugate gebunden werden. Die Immobilisierung der in 200 µl TBS (TBS = 50 mM Tris/HCl, 150 mM NaCl pH 7.5) gelösten Zuckerreste erfolgt unter leichtem Schütteln (4 h bei RT). Dabei wird für eine große Anzahl an unterschiedlichen Saccharidderivaten (geladene und ungeladene Mono- bzw. Oligosaccharide) eine maximale Bindungskapazität von bis zu 20 pmol unter sättigenden Bedingungen (100 pmol Zuckerderivat) gefunden.
Die Detektion der gebundenen Zuckerderivate erfolgt dabei zum einen mit unterschiedlichen Digoxigenin markierten Lectinen (Boehringer Mannheim) und zum anderen über die Bindung eines E-Selectin-Fusionsproteins.

### - Bindungsstudien mit N-Acetyl-neuraminosyl-2,3-D-Lactose:

Als Modellsubstanz zum Testen der Standardbedingungen wird derivatisierte N-Acetyl-neuraminosyl-2,3-D-Lactose in TBS auf eine mit Streptavidin beschichtete Mikrotiterplatte 4 h bei RT inkubiert. Nach Immobilisierung des Zuckerderivats (0.5 - 20 pmol/Vertiefung in der Mikrotiterplatte; gelöst in TBS) werden die Wells 5 x mit 0.25 ml TBS gewaschen. Für die Bindung des Digoxigenin markierten MAA-Lectins (spezifisch für N-Acetyl-neuraminosyl-α(2-3)Gal-Reste) werden 1 - 10 µg des Lectins in 1 ml TBSM (TBSM = TBS mit 1 mM MgCl₂, 1 mM MnCl₂ und 1 mM CaCl₂) mit 0.5 % Blocking Reagenz (Boehringer Mannheim) gelöst und anschließend 0.2 ml der Lectinlösung/Well unter leichtem Schütteln 1 h bei RT inkubiert. Nach der Bindung des Lectins werden die Wells 5 x mit 0.25 ml Puffer (TBST = TBS mit 0.1 % Tween-20® ) gewaschen. Anschließend werden die Wells mit 0.2 ml einer Lösung von Anti-Digoxigenin-POD (0.3 U/ml; Boehringer Mannheim) in TBST unter leichtem Schütteln 1 h bei RT inkubiert. Nach der Bindung des Anti-Digoxigenin-POD werden die Wells 5 x mit TBST gewaschen. Die Detektion erfolgt mit ABTS-Substratlösung für Peroxidase (0.2 ml/Well; Boehringer Mannheim). Die Farbentwicklung wird bei 405 nm und RT nach einer Inkubationszeit von ca. 20 bis 30 min abgelesen. Bei Inkubation von 10 pmol des Zuckerderivats auf der Platte nimmt für das Digoxigenin markierte MAA-Lectin im Bereich von 1 - 5 µg/ml die Signalhöhe linear zu. Beim Einsatz steigender Zuckerderivatmengen (0.5 - 20 pmol) auf der Mikrotiterplatte wird im Bereich von 1 - 10 pmol Saccharid eine lineare Signalzunahme gefunden, wobei die Nachweisgrenze für das N-Acetyl-neuraminosyl-2,3-D-Lactose-derivat bei 0.5 pmol liegt.

### - Bindungsstudien mit biantennärem Undecasaccharid nach Exoglycosidaseverdau:

Nach dem sequentiellen Verdau eines biantennären Undecasaccharidderivats (Beispiel 3) mit den Enzymen Neuramidase (C. perfringens), β-Galactosidase (E. coli); N-Acetyl-β-D-glucosaminidase (Diplococcus pneumoniae) und α-Mannosidase (Jack bean) kann man die einzeln verdauten Saccharidderivate auf einer mit Streptavidin beschichteten Mikrotiterplatte nachweisen. Dazu werden die jeweiligen Oligosaccharidderivate in TBS 4 h bei RT auf eine Mikrotiterplatte immobilisiert. Nach dem Waschen der Platte (5 x 0.25 ml TBS/Well) werden die Wells mit 0.2 ml Lösung der Digoxigenin markierten Lectine SNA-, DSA- (Konzentration 5 µg/ml) und GNA (Konzentration 1 µg/ml) inkubiert (1 h bei RT). Der Nachweis erfolgt anschließend analog der oben beschriebenen Prozedur.

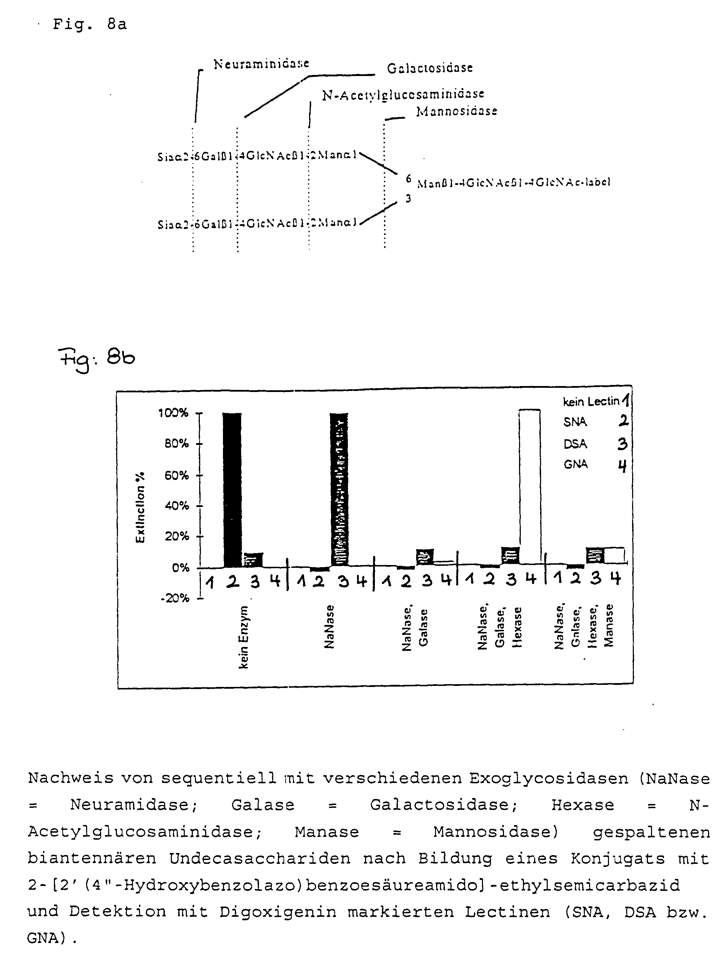

Bei Verdau des in Fig. 8a dargestellten biantennären Undecasaccharids werden deutliche Absorptionen bei Zusatz der Digoxigenin markierten Lectine in drei Fällen erhalten (Fig. 8b) :
- Bei SNA (spezifisch für N-Acetyl-neuraminosyl-α(2-6)Gal-Reste) als Lectin in dem Ansatz mit dem unverdauten Undecasaccharidderivat.
- Bei DSA (spezifisch für Gal(β 1→4)GlcNAc-Reste) als Lectin in dem Ansatz mit dem mittels Neuramidase verdauten Saccharidderivat.
- Bei GNA (spezifisch für Man (β 1→3)Man- bzw. Man (β 1→6)Man-Reste) als Lectin in dem Ansatz mit dem mittels Neuramidase, Galactosidase und N-Acetyl-β-D-glucosaminidase verdauten Saccharidderivat.
Alle anderen Wells sowie die Blindwertkontrollen der Wells ohne Zuckerderivat bzw. ohne Lectin zeigen dagegen Absorptionen unter 5 %, was die Spezifität der Lectine in diesem Test belegt.

Eine direkte Verdauung des biantennären Undecasaccharids kann auch in der Mikrotiterplatte erfolgen. Dazu werden pro Well 20 pmol des Derivats 4 h bei RT immobilisiert und anschließend 1 h bei 37°C mit den vorher beschriebenen Enzymen direkt in der Platte verdaut. Die Detektion der Zuckerreste erfolgt analog der vorher beschriebenen Methode. Das Ergebnis ist mit dem vorhergehenden Test weitgehend identisch.

### - Bindungsstudien mit Oligosacchariden von Glykoproteinen:

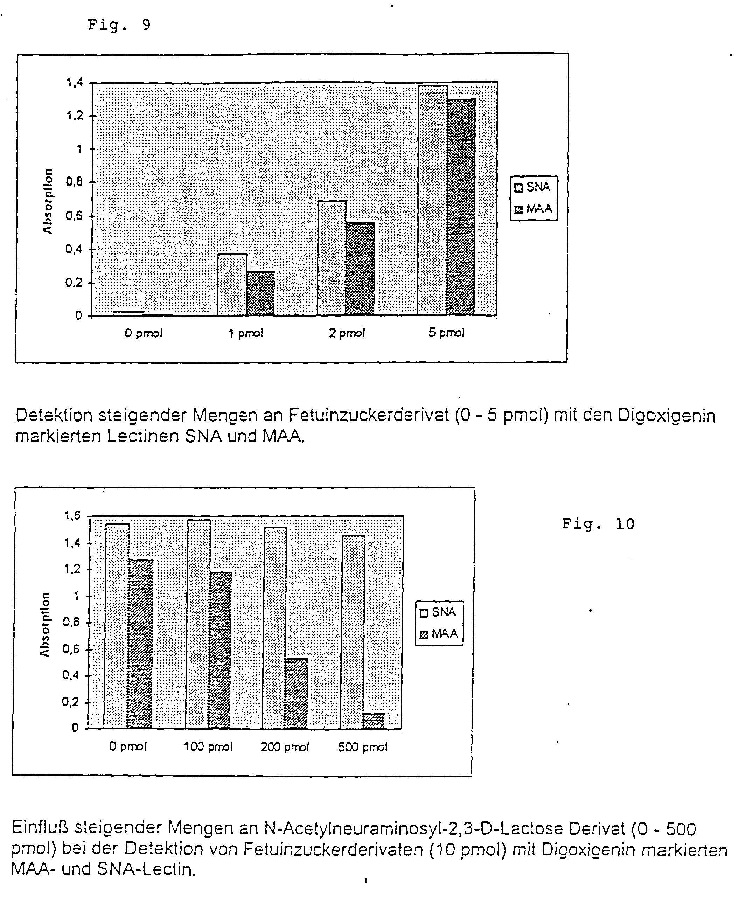

Die durch N-Glycosidase F abgespaltenen Oligosaccharide von Fetuin (Beispiel 3) besitzen Sialinsäurereste an den nichtreduzierenden Enden, deren Bindungstypen α2-6 bzw. α2-3 mittels den Digoxigenin markierten Lectinen SNA (spezifisch für N-Acetyl-neuraminosyl-α(2-6)Gal-Reste) und MAA (spezifisch für N-Acetyl-neuraminosyl-α(2-3)Gal-Reste) unterschieden werden können.
Dazu werden je 0 - 5 pmol der Saccharidderivate in je ein Well einer mit Streptavidin beschichteten Mikrotiterplatte immobilisiert. Die Detektion erfolgt mit je 5 µg/ml Digoxigenin markiertem Lectin in TBSM wie zuvor beschrieben. Fig. 9 zeigt das Ergebnis dieses Versuchs am Beispiel von Fetuinzuckerderivat. Beide Lectine zeigen deutliche Signale, die mit steigender Konzentration des Saccharids in den Wells linear zunehmen. Die gefundene annähernde Gleichverteilung von α2-6 bzw. α2-3 gebundenen Sialinresten entspricht den in der Literatur beschriebenen Werten (Green E. D., Adelt G., Baenzinger J. U., Wilson S. & Van Halbeek H., J. Biol. Chem., 263 (1988),18253 - 68).

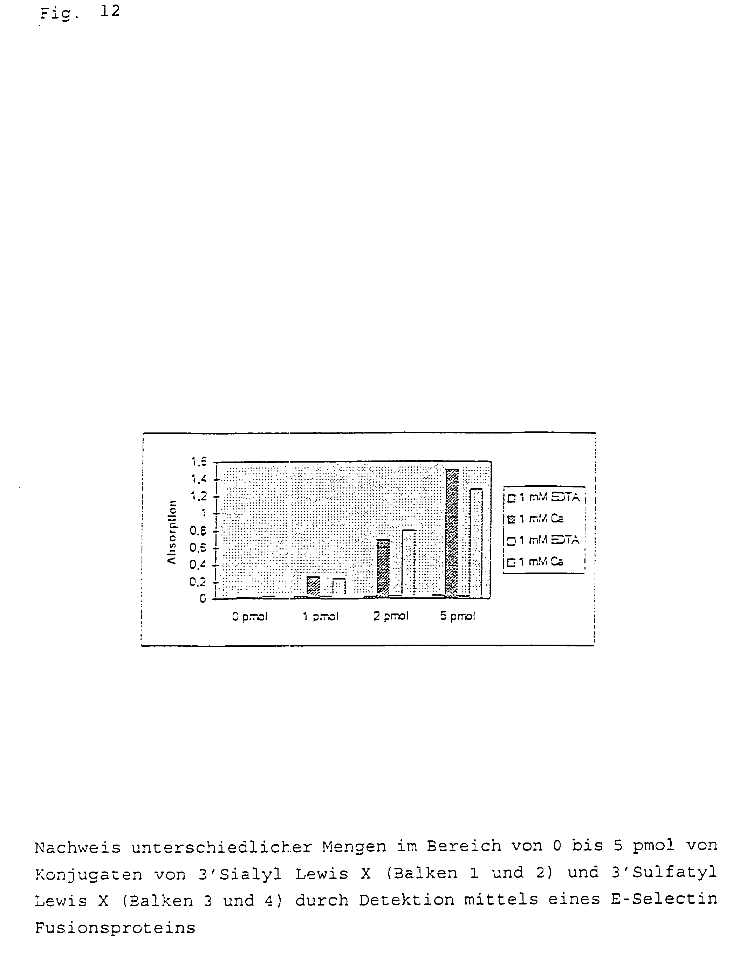

Bei der spezifischen Inhibierung des Digoxigenin markierten MAA-Lectins durch Zugabe von N-Acetyl-neuraminosyl-2,3-D-Lactose bei der Lectininkubation von Fetuinzuckerderivaten (10 pmol) auf der MTP kann bei 10- bis 50-fachem Zuckerüberschuß in den Wells eine Abnahme der Signalstärke beobachtet werden (Fig. 10). Die Signalstärke beträgt etwa 50% des Wertes ohne Inhibierung bei Zugabe von 200 pmol N-Acetyl-neuraminosyl-2,3-D-Lactose und etwa 15% bei Zugabe von 500 pmol N-Acetyl-neuraminosyl-2,3-D-Lactose. In den Wells mit SNA als Lectin tritt wie erwartet keine Inhibition durch die Zugabe des Saccharids auf.
Bei spezifischer Abspaltung der α2-3 gebundenen N-Acetyl-neuraminsäure von den Fetuinzuckerderivaten mittels NDV-Sialidase von Boehringer Mannheim (20 mU; 1 h bei 37°C) kann in den Wells mit MAA als Lectin keine Absorption mehr beobachtet werden.
Die Messungen mit Digoxigenin markierten SNA als Lectin zeigen dagegen nahezu dieselben Absorptionen wie die unbehandelten Fetuinzuckerderivate (Fig. 11).

Analoge Ergebnisse werden bei der Detektion der Transferrinzuckerderivate mit Digoxigenin markiertem SNA Lectin (Konzentration 5 µg/ml) und der Ribonuklease B Zuckerderivate mit Digoxigenin markiertem GNA Lectin (Konzentration 1 µg/ml) erhalten.

### - Bindungsstudien von E-Selectin mit von 3'-Sialyl Lewis X und 3'-Sulfatyl Lewis X:

Die beiden Oligosaccharide 3'-Sialyl Lewis X und 3'-Sulfatyl Lewis X (je 1 - 5 pmol/Well; Oxford Glyco Systems) werden als Konjugat an eine mit Streptavidin beschichtetete Mikrotiterplatte gebunden. Nach Bindung des Zuckerkonjugats werden die Wells 5 x mit 0.25 ml TBST gewaschen und die Wells anschließend mit 1 %iger BSA-Lösung (1 h bei RT) blockiert. Nach Entfernung der Lösung werden 0.5 µg eines E-Selectin Fusionsproteins in die Wells gebunden (soluble E-Selectin fusioniert an die κ-light chain exprimiert in SF-9 Zellen). Nach der Bindung des Rezeptors und 5 maligem Waschen der Wells mit TBST erfolgt die Bindung von Anti-Maus-Ig-POD (0.3 U/ml; Boehringer Mannheim) in TBST. Nach dem Waschen mit TBST erfolgt die Detektion wie zuvor beschrieben.
Im Bereich von 1 - 5 pmol der Saccharidderivate steigen die Signale für beide Derivate linear an (Fig. 12). Die Bindung des E-Selectins an die Zuckerderivate ist dabei strikt abhängig von der Zugabe von Ca²⁺-Ionen (Zugabe von 1 mM CaCl₂ zu dem Puffer). Die Blindwertkontrollen der Wells ohne Zuckerderivat bzw. ohne E-Selectin bzw. bei Komplexierung des Ca²⁺ durch EDTA (1 mM in der Platte) ergeben keine meßbaren Absorptionen.

### Beispiel 5:

### Derivatisierung von N-Acetyl-neuraminosyl-2,3-D-Lactose mit Biotinyl-L-3-(2-Naphtyl)-alaninhydrazid (BNAH)

### a) Umsetzungen:

Für die Umsetzung von N-Acetyl-neuraminosyl-2,3-D-Lactose für analytische Zwecke werden in einem verschließbaren Eppendorfcap 1 nmol des Saccharids mit dem fünffachen molaren Überschuß an Biotinyl-L-3-(2-Naphthyl)-alaninhydrazid (Boehringer Mannheim) in 100 µl MeOH/AcOH (9:1; v:v) 6 h bei 60°C umgesetzt. Nach der Umsetzung wird auf der HPLC (ODS-Hypersil; Eluent A: 0.1 % TFA in H₂O; Eluent B: 0.1 % TFA in CH₃CN; Gradient 0 - 50 % in 30 min) nur ein Signal des umgesetzen Zuckerderivats beobachtet. Auf der DC (Laufmittel: MeOH:H₂O:Butan-2-on = 6:2:2; v:v:v; Detektion der Zucker mittels 20 % H₂SO₄ in EtOH) konnte nach der Derivatisierung kein freier Zucker mehr nachgewiesen werden, woraus auf eine Umsetzungsrate von > 95 % geschlossen werden kann. Die Nachweisgrenze der Saccharidderivate mittels Fluoreszenzdetektion (λₑₓ = 275 nm und λₑₘ = 324 nm) liegt bei ca. 10 pmol. Der Marker BNAH eluiert bei ca. 35 % CH₃CN von der RP18-Säule und kann so gut von den Konjugaten getrennt werden, die bei 15 - 25 % CH₃CN eluieren.

### b) Stabilitäten:

Die Saccharidderivate zeigen in Lösung bei 25°C (40 h) bzw. nach Lyophylisation keine Zersetzungserscheinungen, was durch HPLC-Analyse bestätigt wurde.
Abtrennung des Farbstoff-Überschusses:
Die Abtrennung des BNAH-Überschusses nach Umsetzung mit den Sacchariden erfolgt durch Extraktion mit Essigsäureethylester. Dazu werden zu den 100 µl Ansätzen 100 µl H₂O bidest. und 800 µl Essigsäureethylester gegeben; nach Durchmischen und Zentrifugation (1 min bei 10000 g) wird die obere Essigesterphase enffernt und die Extraktion mit der unteren wässrigen Phase noch zweimal wiederholt. Dabei können 90 % des BNAH abgetrennt werden. Der Verlust an Zuckerderivat, getestet mit einem Glucosederivat, liegt unterhalb der Nachweisqrenze von 5 %.

### Beispiel 6:

### Bindungsstudien mit N-Acetyl-neuraminosyl-2,3-D-Lactose auf Streptavidin-Mikrotiterplatten.

Auf mit Streptavidin beschichteten Mikrotiterplatten können die derivatisierten Saccharide, gelöst in 200 µl TBS, unter leichtem Schütteln (2 h bei RT) gebunden werden.
Die maximale Bindungskapazität beträgt bis zu 20 pmol Derivat/Well unter sättigenden Bedingungen (100 pmol Zuckerderivat).
Für die Detektion von N-Acetyl-neuraminosyl-2,3-D-Lactose werden nach Immobilisierung des Zuckerderivats (0.5 - 20 pmol/Vertiefung in der Mikrotiterplatte; gelöst in TBS) die Wells 5 x mit 0.25 ml TBS gewaschen. Für die Bindung des Digoxigenin markierten MAA-Lectins (spezifisch für N-Acetyl-neuraminosyl-α(2-3)Gal-Reste) werden 1 - 10 µg des Lectins in 1 ml TBSM mit 0.5 % Blocking Reagenz gelöst und anschließend 0.2 ml der Lectinlösung/Well unter leichtem Schütteln 1 h bei RT inkubiert. Nach der Bindung des Lectins werden die Wells 5 x mit 0.25 ml TBST-Puffer gewaschen. Anschließend werden die Wells mit 0.2 ml einer Lösung von Anti-Digoxigenin-POD (0.3 U/ml) in TBST unter leichtem Schütteln 1 h bei RT inkubiert. Nach der Bindung des Anti-Digoxigenin-POD werden die Wells 5 x mit TBST gewaschen. Die Detektion erfolgt mit 0.2 ml ABTS-Substratlösung/Well für Peroxidase (0.3 g/l). Die Farbentwicklung wird bei 405 nm und RT nach einer Inkubationszeit von ca. 20 bis 30 min abgelesen. Bei Einsatz steigender Zuckerderivatmengen (0.5 - 20 pmol) auf der Mikrotiterplatte wird im Bereich von 1 - 10 pmol eine lineare Signalzunahme gefunden, wobei die Nachweisgrenze für das N-Acetyl-neuraminosyl-2,3-D-Lactose-derivat bei 0.5 pmol liegt.

## Patentansprüche

1. Verbindung mit einer Struktur der Formel (I):
X - Y (I)
**dadurch gekennzeichnet,**
**daß** X einen Rest darstellt ausgewählt aus -NR-NH₂, -C(A)-NR-NH₂ oder -B-C(A)-NR-NH₂, wobei R Wasserstoff oder ein Alkylrest mit 1-4 C-Atomen ist und A und B jeweils unabhängig NH, O oder S darstellen und Y einen Rest darstellt, der einen fluoreszierenden Chromophor oder einen Chromophor, welcher ein Absorptionsmaximum bei einer Wellenlänge λₘₐₓ von mehr als 250 nm oder/und bei der Wellenlänge λₘₐₓ des Absorptionsmaximums einen molaren Extinktionskoeffizienten ∈ von mehr als 1000 1 mol⁻¹ cm⁻¹ aufweist, sowie einen an Streptavidin oder/und Avidin bindefähigen Liganden enthält.

2. Verbindung nach Anspruch 1,
**dadurch gekennzeichnet,**
**daß** der an Avidin oder/und Streptavidin bindefähige Ligand Biotin oder ein Biotinderivat, insbesondere Iminobiotin, Diaminobiotin und Desthiobiotin, darstellt.

3. Verbindung nach Anspruch 1,
**dadurch gekennzeichnet,**
**daß** der an Avidin oder/und Streptavidin bindefähige Ligand selbst ein Chromophor ist.

4. Verbindung nach Anspruch 3,
**dadurch gekennzeichnet,**
**daß** die Verbindung X-Y eine Struktur der Formel (II) aufweist: 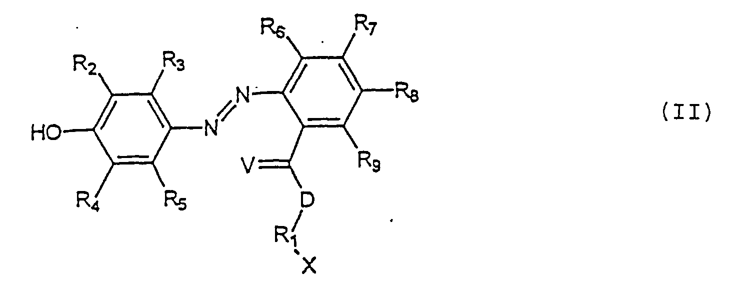 worin
X wie in Anspruch 1 definiert ist, V und D jeweils unabhängig NH, O oder S darstellen,
R₁ eine gegebenenfalls Heteroatome enthaltende Alkylen-, Alkenylen- oder Alkinylengruppe mit einer Länge von 2 bis 20 Atomen darstellt und
R₂ bis R₉ jeweils unabhängig Wasserstoff oder eine Alkylgruppe mit 1 bis 4 Kohlenstoffatomen darstellen.

5. Verbindung nach Anspruch 4,
**dadurch gekennzeichnet,**
**daß** X-Y 2-[2'-(4''-Hydroxybenzolazo)-benzosäureamido]ethylsemicarbazid darstellt.

6. Verbindung nach einem der Ansprüche 1 bis 5,
**dadurch gekennzeichnet,**
**daß** X einen Rest -B-C(A)-NR-NH₂ oder NR-NH₂ darstellt, worin A, B und R wie in Anspruch 1 definiert sind.

7. Verbindung nach einem der Ansprüche 1 bis 2,
**dadurch gekennzeichnet,**
**daß** Y eine Struktur der Formel (IV) 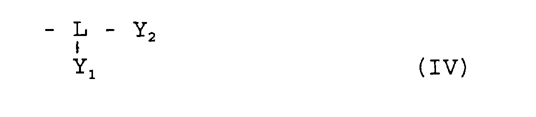 aufweist, worin
L eine lineare, gegebenenfalls Heteroatome enthaltende Verknüpfungsgruppe mit einer Kettenlänge von 1 bis 10 Atomen, darstellt,
Y₁ eine Gruppe ist, die einen Chromophor enthält und als Seitengruppe an L gebunden ist und
Y₂ eine an Streptavidin oder/und Avidin bindefähige Gruppe enthält.

8. Verbindung nach Anspruch 7,
**dadurch gekennzeichnet,**
**daß** Y die Gruppe 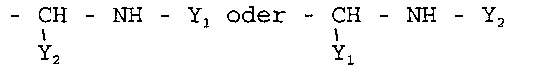 bedeutet, worin Y₁ einen Rest darstellt, der einen Chromphor enthält und Y₂ einen Rest darstellt, der einen an Streptavidin oder/und Avidin bindefähigen Liganden enthält.

9. Verbindung nach Anspruch 8,
**dadurch gekennzeichnet,**
**dass** Y₂ ausgewählt ist aus den Gruppen -((CH₂)ₙV_{q})-K und - (C(O) - (CH₂)ₙV)_{q}-K, worin V NH, O oder S darstellt,
K Biotin oder ein Biotinderivat ist,
n = O bis 20, q = O oder 1 ist
sowie Y₁ eine Gruppe -CHR- (CH₂)ₙ-Q darstellt, worin n=0 bis 20, R Wasserstoff oder eine Alkylgruppe ist und Q einen aromatischen oder heteroaromatischen Rest darstellt.

10. Verbindung nach Anspruch 9,
**dadurch gekennzeichnet,**
**daß** X-Y Biotinyl-L-3-(2-Naphthyl)alaninhydrazid ist.

11. Verwendung einer Verbindung nach einem der Ansprüche 1 bis 10, als Reagenz zur Derivatisierung einer eine Aldehyd-, Keton-, Semiketal oder Semiacetalgruppe enthaltenden Substanz.

12. Verwendung nach Anspruch 11 als Reagenz zur Derivatisierung von Sacchariden.

13. Konjugat gebildet aus einer eine Aledhyd-, Keton-, Semiketal- oder Semiacetal-Gruppe enthaltenden Substanz und einer Verbindung der Formel (I) nach einem der Ansprüche 1 bis 10.

14. Konjugat nach Anspruch 13, das durch Reaktion zwischen der Gruppe X und der Aldehyd-, Keton, Semiketal- oder Semiacetal-Gruppe gebildet ist.

15. Konjugat nach einem der Ansprüche 13 bis 14,
**dadurch gekennzeichnet,**
**daß** die eine Aldehyd-, Keton-, Semiacetal- oder Semiketal-Gruppe enthaltende Verbindung ein Saccharid, insbesondere ein Mono-, Di- oder Oligosaccharid ist.

16. Verfahren zur Herstellung eines Konjugats nach einem der Ansprüche 13 bis 15,
**dadurch gekennzeichnet,**
**daß** eine eine Aldehyd-, Keton-, Semiketal- oder Semiacetal-Funktion enthaltende Verbindung mit einer Verbindung der Formel (I) nach einem der Ansprüche 1 bis 10 unter Bedingungen umgesetzt wird, bei denen die Gruppe X der Verbindung (I) mit der Aldehyd-, Keton-, Semiketal- oder Semiacetal-Gruppe reagiert.

17. Komplex gebildet aus Avidin oder Streptavidin und einem Konjugat nach einem der Ansprüche 13 bis 15.

18. Komplex nach Anspruch 17,
**dadurch gekennzeichnet,**
**daß** 1 bis 4 Moläquivalente eines Konjugats nach einem der Ansprüche 13 bis 15 pro Moläquivalent Avidin oder Streptavidin vorhanden sind.

19. Komplex nach Anspruch 17 oder 18,
**dadurch gekennzeichnet,**
**daß** das Avidin oder Streptavidin immobilisiert an einer Festphase vorliegt.

20. Verwendung eines Komplexes nach einem der Ansprüche 17 bis 19 als Immunogen zur Herstellung von Anti-Glycan-Antikörpern.

21. Verfahren zum Nachweis oder/und zur Isolierung von Kohlehydraten, umfassend die Schritte:
(a) Inkontaktbringen einer Verbindung nach einem der Ansprüche 1 bis 10 mit einer ein nachzuweisendes oder/und zu isolierendes Kohlehydrat enthaltenden Probe,
(b) Bildung eines Konjugats aus nachzuweisendem Kohlehydrat und der Verbindung und
(c) Nachweis oder/und Isolierung des Konjugats.

22. Verfahren nach Anspruch 21,
**dadurch gekennzeichnet,**
das die Verbindungen (i) zu den Konjugaten umgesetzt und (ii) danach durch Inkubation mit Streptavidin oder/und Avidin in einen Komplex überführt werden.

23. Verfahren nach Anspruch 21,
**dadurch gekennzeichnet,**
**daß** die Verbindungen (i) durch Inkubation mit Streptavidin oder/und Avidin in einen Komplex überführt und (ii) danach zu den Konjugaten umgesetzt werden.

24. Verfahren nach Anspruch 22 oder 23,
**dadurch gekennzeichnet,**
**daß** das Avidin oder/und Streptavidin an eine Festphase gebunden wird und das Kohlehydrat über seine Bindung an die Festphase nachgewiesen oder/und isoliert wird.

25. Verfahren nach einem der Ansprüche 21 bis 24,
**dadurch gekennzeichnet,**
**daß** der Nachweis der Kohlehydrate durch Bindung eines Glycanrezeptors an das Konjugat erfolgt.

26. Verfahren nach Anspruch 25,
**dadurch gekennzeichnet,**
**daß** der Glycanrezeptor direkt oder indirekt markiert ist.

27. Verfahren zum Nachweis oder/und zur Isolierung von Glycanrezeptoren, umfassend die Schritte
(a) Bildung von Konjugaten aus einem oder mehreren ausgewählten Kohlehydraten und einer Verbindung der Ansprüche 1 bis 10,
(b) Inkontaktbringen des Konjugats mit einer einen nachzuweisenden oder/und zu isolierenden Glycanrezeptor enthaltenden Probe und
(c) Nachweis oder/und Isolierung des Glycanrezeptors.

28. Verfahren nach Anspruch 27,
**dadurch gekennzeichnet,**
**daß** die Konjugate (i) durch Inkubation mit Streptavidin oder/und Avidin in einem Komplex überführt und (ii) danach mit der Probe in Kontakt gebracht werden.

29. Verfahren nach Anspruch 28,
**dadurch gekennzeichnet,**
**daß** die Konjugate (i) mit der Probe in Kontakt gebracht und (ii) danach durch Inkubation mit Streptavidin oder/und Avidin in einen Komplex überführt werden.

30. Verfahren nach Anspruch 28 oder 29,
**dadurch gekennzeichnet,**
**daß** das Steptavidin oder/und Avidin an eine Festphase gebunden wird und der Glycanrezeptor über seine Bindung an die Festphase nachgewiesen oder/und isoliert wird.

31. Reagenzienkit zum Nachweis oder/und zur Isolierung von Kohlehydraten, umfassend:
(a) eine Verbindung nach einem der Ansprüche 1 bis 10,
(b) eine mit Streptavidin oder/und Avidin beschichtete Festphase.

32. Reagenzienkit nach Anspruch 31,
weiterhin umfassend
(c) mindestens ein Kohlehydrat-spaltendes Enzym.

33. Reagenzienkit zum Nachweis oder/und zur Isolierung von Glycanrezeptoren, umfassend:
(a) eine Verbindung nach einem der Ansprüche 1 bis 10, die gegebenenfalls in Form eines Konjugats nach einem der Ansprüche 13 bis 15 vorliegen kann und
(b) eine mit Streptavidin oder/und Avidin beschichtete Festphase.

34. Verbindung der Formel (III): 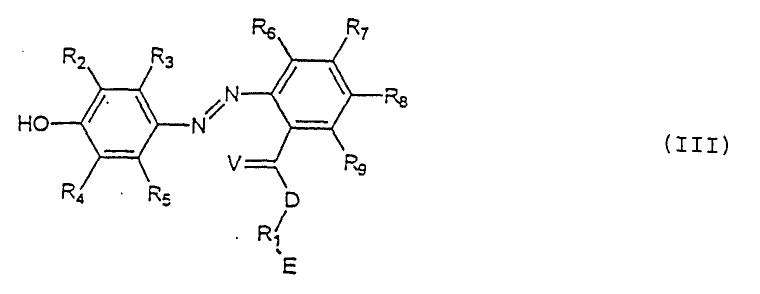 worin V, D und R₁ bis R₉ wie in Anspruch 6 definiert sind und E OH, SH, NH₂ oder COOH bedeutet.

35. Verwendung einer Verbindung nach Anspruch 34 zur Herstellung von Verbindungen der Formel (II).

## Claims

1. Compound having a structure of the formula (I):
X - Y (I)
**wherein**
X represents a residue selected from -NR-NH₂, -C(A)-NR-NH₂ or -B-C(A)-NR-NH₂ in which R is hydrogen or an alkyl residue with 1 to 4 C atoms and A and B each independently represent NH, O or S and Y represents a residue which contains a fluorescent chromophore or a chromophore that has an absorption maximum at a wavelength λₘₐₓ of more than 250 nm or/and has a molar extinction coefficient ε of more than 1000 1 mol⁻¹ cm⁻¹ at the wavelength λₘₐₓ of the absorption maximum as well as a ligand capable of binding to streptavidin or/and avidin.

2. Compound as claimed in claim 1,
**wherein**
the ligand capable of binding to avidin or/and streptavidin is biotin or a biotin derivative in particular iminobiotin, diaminobiotin and desthiobiotin.

3. Compound as claimed in claim 1,
**wherein**
the ligand capable of binding to avidin or/and streptavidin is itself a chromophore.

4. Compound as claimed in claim 3,
**wherein**
the compound X - Y has a structure of formula (II) : 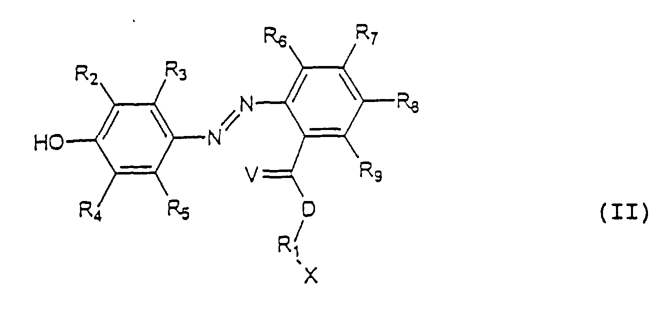 in which
X is defined as in claim 1, V and D each independently represent NH, O or S,
R₁ represents an alkylene, alkenylene or alkinylene group optionally containing heteroatoms with a length of 2 to 20 atoms and
R₂ to R₉ each independently denote hydrogen or an alkyl group with 1 to 4 carbon atoms.

5. Compound as claimed in claim 4,
**wherein**
X-Y represent a 2-[2'-(4''-hydroxybenzeneazo)benzoic acid-amido]ethyl semicarbazide.

6. Compound as claimed in one of the claims 1 - 5,
**wherein**
X represents a residue -B-C(A)-NR-NH₂ or NR-NH₂ in which A, B and R are defined as in claim 1.

7. Compound as claimed in one of the claims 1 to 2,
**wherein**
Y has a structure of formula (IV) 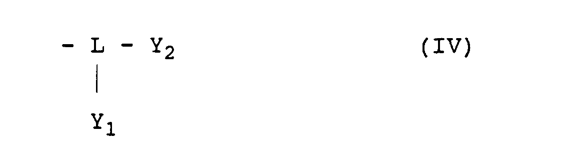 in which
L is a linear linker group L optionally containing heteroatoms with a chain length of 1 to 10 atoms
Y₁ is a group which contains a chromophore and is bound as a side group to L and
Y₂ contains a group capable of binding to streptavidin or/and avidin.

8. Compound as claimed in claim 7,
**wherein**
Y denotes the group 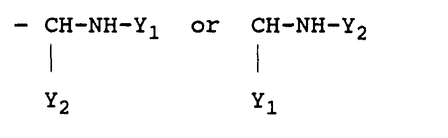 in which Y₁ represents a residue which contains a chromophore and Y₂ represents a residue which contains a ligand capable of binding to streptavidin or/and avidin.

9. Compound as claimed in claim 8,
**wherein**
Y₂ is selected from the groups -((CH₂)ₙV_{q})-K, -C(O)-(CH₂)ₙV)_{q}-K in which V represents NH, O or S,
K is biotin or a biotin derivative,
n = 0 to 20, q = 0 or 1,
and Y₁ represents a group CHR-(CH₂)ₙ-Q in which n = 0 to 20, R is hydrogen or an alkyl group and Q represents an aromatic or heteroaromatic residue.

10. Compound as claimed in claim 9,
**wherein**
X-Y is biotinyl-L-3-(2-naphthyl)alanine hydrazide.

11. Use of a compound as claimed in one of the claims 1 - 10 as a reagent for the derivatization of a substance containing an aldehyde, ketone, semiketal or semiacetal group.

12. Use as claimed in claim 11 as a reagent for the derivatization of saccharides.

13. Conjugate formed from a substance containing an aldehyde, ketone, semiketal or semiacetal group and a compound of formula (I) as claimed in one of the claims 1 - 10.

14. Conjugate as claimed in claim 13, which is formed by reaction between the group X and the aldehyde, ketone, semiketal or semiacetal group.

15. Conjugate as claimed in one of the claims 13 - 14,
**wherein**
the compound containing an aldehyde, ketone, semiacetal or semiketal group is a saccharide and in particular a monosaccharide, disaccharide or oligosaccharide.

16. Process for the production of a conjugate as claimed in one of the claims 13 to 15,
**wherein**
a compound containing an aldehyde, ketone, semiketal or semiacetal group is reacted with a compound of formula (I) as claimed in one of the claims 1 to 10 under conditions such that the group X of compound (I) reacts with the aldehyde, ketone, semiketal or semiacetal group.

17. Complex formed from avidin or streptavidin and a conjugate as claimed in one of the claims 13 to 15.

18. Complex as claimed in claim 17,
**wherein**
1 - 4 mole equivalents of a conjugate as claimed in claims 13 to 15 are present per mole equivalent avidin or streptavidin.

19. Complex as claimed in claim 17 or 18,
**wherein**
avidin or streptavidin is present immobilized on a solid phase.

20. Use of a complex as claimed in one of the claims 17 to 19 as an immunogen for the production of anti-glycan antibodies.

21. Method for the detection or/and isolation of carbohydrates comprising the steps:
(a) contacting a compound as claimed in one of the claims 1 to 10 with a sample containing a carbohydrate to be detected or/and isolated,
(b) forming a conjugate from the carbohydrate to be detected and the compound and
(c) detecting or/and isolating the conjugate.

22. Method as claimed in claim 21,
**wherein**
the compounds are (i) reacted to form the conjugates and (ii) afterwards they are converted into a complex by incubation with streptavidin or/and avidin.

23. Method as claimed in claim 21,
**wherein**
the compounds are (i) converted into a complex by incubation with streptavidin or/and avidin and (ii) afterwards reacted to form the conjugates.

24. Method as claimed in claim 22 or 23,
**wherein**
the avidin or/and streptavidin is bound to a solid phase and the carbohydrate is detected or/and isolated by means of its binding to the solid phase.

25. Method as claimed in one of the claims 21 to 24,
**wherein**
the carbohydrates are detected by binding a glycan receptor to the conjugate.

26. Method as claimed in claim 25,
**wherein**
the glycan receptor is labelled directly or indirectly.

27. Method for the detection or/and isolation of glycan receptors comprising the steps:
(a) forming conjugates from one or several selected carbohydrates and a compound as claimed in claims 1 to 10
(b) contacting the conjugate with a sample containing a glycan receptor to be detected or/and isolated and
(c) detecting or/and isolating the glycan receptor.

28. Method as claimed in claim 27,
**wherein**
the conjugates are (i) converted into a complex by incubation with streptavidin or/and avidin and (ii) subsequently contacted with the sample.

29. Method as claimed in claim 28,
**wherein**
the conjugates are (i) contacted with the sample and (ii) subsequently converted into a complex by incubation with streptavidin or/and avidin.

30. Method as claimed in claim 28 or 29,
**wherein**
the streptavidin or/and avidin is bound to a solid phase and the glycan receptor is detected or/and isolated by means of its binding to the solid phase.

31. Reagent kit for the detection or/and isolation of carbohydrates comprising:
(a) a compound as claimed in one of the claims 1 to 10
(b) a solid phase coated with streptavidin or/and avidin.

32. Reagent kit as claimed in claim 31,
additionally comprising
(c) at least one carbohydrate-cleaving enzyme.

33. Reagent kit for the detection or/and isolation of glycan receptors comprising:
(a) a compound as claimed in one of the claims 1 to 10 which can optionally be present in the form of a conjugate as claimed in one of the claims 13 to 15 and
(b) a solid phase coated with streptavidin or/and avidin.

34. Compound of formula (III): 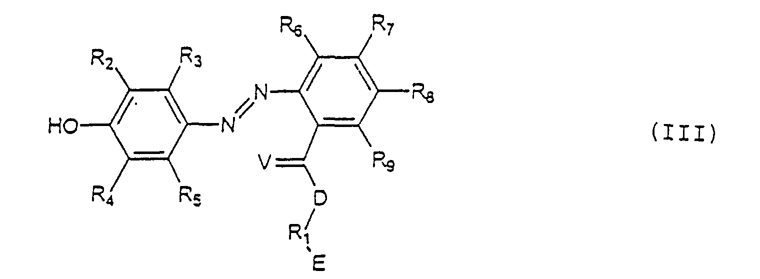 in which V, D and R₁ to R₉ are defined as in claim 6 and E denotes OH, SH, NH₂ or COOH.

35. Use of a compound as claimed in claim 34 for the production of compounds of formula (II).

## Revendications

1. Composé ayant une structure de la formule (I) :
X - Y (I)
**caractérisé en ce que**
X représente un reste choisi à partir de -NR-NH₂, -C(A)-NR-NH₂ ou -B-C(A)-NR-NH₂, R étant l'hydrogène ou un reste alkyle avec 1-4 atomes C, et A et B représentent respectivement, de façon indépendante, NH, O ou S, et Y représente un reste qui présente un maximum d'absorption pour une longueur d'onde λₘₐₓ de plus de 250 nm ou/et pour la longueur d'onde λₘₐₓ du maximum d'absorption un coefficient d'extinction molaire ε supérieur à 1000 1 mol⁻¹ cm⁻¹ ainsi qu'un ligand pouvant se fixer sur la streptavidine ou/et l'avidine.

2. Composé selon la revendication 1, **caractérisé en ce que** le ligand pouvant se fixer sur l'avidine ou/et la streptavidine représente la biotine ou un dérivé de biotine, en particulier l'iminobiotine, la diaminobiotine et la desthiobiotine.

3. Composé selon la revendication 1, **caractérisé en ce que** le ligand pouvant se fixer sur l'avidine ou/et la streptavidine est lui-même un chromophore.

4. Composé selon la revendication 3, **caractérisé en ce que** le composé X-Y présente une structure de formule (II) : 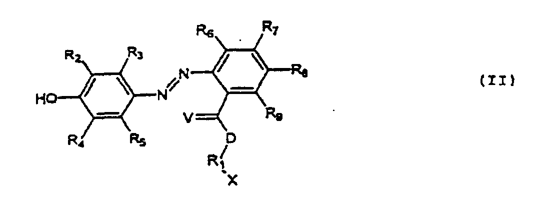 dans laquelle
X est défini comme dans la revendication 1, V et D représentent respectivement, de façon indépendante, NH, O ou S,
R₁ représente un groupe alkylène, alcényle ou alkinylène contenant éventuellement des hétéroatomes d'une longueur de 2 à 20 atomes, et
R₂ à R₉ représentent un groupe alkyle ou un hydrogène respectivement indépendant avec 1 à 4 atomes de carbone.

5. Composé selon la revendication 4, **caractérisé en ce que** X-Y représente 2-[2'-(4''-hydroxybenzolazo)benzoacide-amido]éthylsemicarbazide.

6. Composé selon l'une des revendications 1 à 5, **caractérisé en ce que** X représente un reste -B-C(A)-NR-NH₂ ou NR-NH₂, dans lequel A, B et R sont tels que définis dans la revendication 1.

7. Composé selon l'une des revendications 1 à 2, **caractérisé en ce que** Y présente une structure de formule (IV) 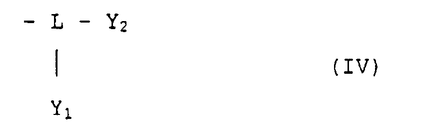 où
L est un groupe de liaison linéaire, contenant éventuellement des hétéroatomes avec une longueur de chaîne de 1 à 10 atomes,
Y₁ est un groupe qui contient un chromophore et qui, en tant que groupe latéral, est fixé sur L, et
Y₂ contient un groupe pouvant être fixé sur streptavidine ou/et avidine.

8. Composé selon la revendication 7, **caractérisé en ce que** Y signifie le groupe 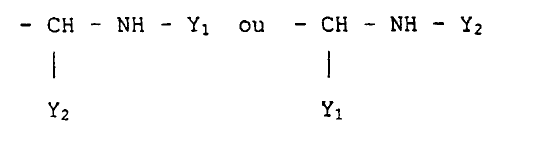 où Y₁ représente un reste qui contient un chromophore et Y₂ représente un reste qui contient un ligand pouvant être fixé sur streptavidine ou/et avidine.

9. Composé selon la revendication 8, **caractérisé en ce que** Y₂ est choisi à partir des groupes -((CH₂)ₙV_{q})-K et -(C(O) - (CH₂)ₙV)_{q}-K, où V représente NH, O ou S,
K est biotine ou un dérivé de biotine,
n = 0 jusqu'à 20, q = 0 ou 1,
ainsi que Y₁ représente un groupe -CHR- (CH₂)ₙ-Q, où n = 0 jusqu'à 20, R est l'hydrogène ou un groupe alkyle et Q représente un radical aromatique ou hétéroaromatique.

10. Composé selon la revendication 9, **caractérisé en ce que** X-Y est biotinyl-L-3-(2-naphtyl)alaninhydrazide.

11. Utilisation d'un composé selon l'une des revendications 1 à 10, en tant que réactif pour la formation de dérivés d'une substance contenant un groupe aldéhyde, cétone, semicétal ou semiacétal.

12. Utilisation selon la revendication 11 en tant que réactif pour la formation de dérivés de saccharide.

13. Conjugué formé à partir d'une substance contenant un groupe aldéhyde, cétone, semicétal ou semiacétal et un composé de formule (I) selon l'une des revendications 1 à 10.

14. Conjugué selon la revendication 13 qui est formé par réaction entre le groupe X et le groupe aldéhyde, cétone, semicétal ou semiacétal.

15. Conjugué selon l'une des revendications 13 à 14, **caractérisé en ce que** le composé contenant le groupe aldéhyde, cétone, semiacétal ou semicétal est un saccharide, en particulier un mono-, di- ou oligosaccharide.

16. Procédé pour la préparation d'un conjugué selon l'une des revendications 13 à 15, **caractérisé en ce que** l'on met en réaction un composé contenant une fonction aldéhyde, cétone, sémicétal ou semiacétal avec un composé de la formule (I) selon l'une des revendications 1 à 10, dans les conditions dans lesquelles le groupe X fait réagir le composé (I) avec le groupe aldéhyde, cétone, semicétal ou semiacétal.

17. Complexe formé à partir d'avidine ou de streptavidine et d'un conjugué selon l'une des revendications 13 à 15.

18. Complexe selon la revendication 17, **caractérisé en ce que** sont présents 1 à 4 équivalents molaires d'un conjugué selon l'une des revendications 13 à 15 par équivalent molaire d'avidine ou de streptavidine.

19. Complexe selon la revendication 17 ou 18, **caractérisé en ce que** l'avidine ou la streptavidine est immobilisée sur une phase solide.

20. Utilisation d'un complexe selon l'une des revendications 17 à 19 en tant qu'immunogène pour la préparation d'anticorps antiglycane.

21. Procédé pour la détection ou/et l'isolation d'hydrate de carbone, comprenant les étapes consistant à :
(a) mettre en contact un composé selon l'une des revendications 1 à 10 avec un échantillon contenant un hydrate de carbone à détecter ou/et à isoler,
(b) formation d'un conjugué à partir d'hydrate de carbone à détecter et le composé, et
(c) détection ou/et isolation du conjugué.

22. Procédé selon la revendication 21, **caractérisé en ce que** les composés (i) sont mis en réaction avec les conjugués, et (ii) ensuite, par incubation avec la streptavidine ou/et l'avidine, transformés en un complexe.

23. Procédé selon la revendication 21, **caractérisé en ce que** les composés (i) sont transformés par incubation avec la streptavidine ou/et l'avidine en un complexe, et (ii) sont ensuite transformés en conjugués.

24. Procédé selon la revendication 22 ou 23, **caractérisé en ce que** l'avidine ou/et la streptavidine sont fixées sur une phase solide et l'hydrate de carbone est détecté ou est isolé sur sa fixation sur la phase solide.

25. Procédé selon l'une des revendications 21 à 24, **caractérisé en ce que** la détection des hydrates de carbone s'effectue par fixation d'un récepteur de glycane sur le conjugué.

26. Procédé selon la revendication 25, **caractérisé en ce que** le récepteur glycane est marqué directement ou indirectement.

27. Procédé pour la détection ou/et l'isolation de récepteur de glycane, comprenant les étapes consistant à :
(a) former des conjugués à partir d'un ou de plusieurs hydrates de carbone choisis et d'un composé des revendications 1 à 10,
(b) mise en contact du conjugué avec un échantillon contenant un récepteur de glycane à détecter ou/et à isoler,
(c) détection ou/et isolation du récepteur de glycane.

28. Procédé selon la revendication 27, **caractérisé en ce que** les conjugués (i) sont transformés par incubation avec la streptavidine ou/et l'avidine en un complexe, et (ii) sont ensuite mis en contact avec l'échantillon.

29. Procédé selon la revendication 28, **caractérisé en ce que** les conjugués (i) sont mis en contact avec l'échantillon, et (ii) sont ensuite transformés par incubation avec la streptavidine ou/et avidine transformés en un complexe.

30. Procédé selon la revendication 28 ou 29, **caractérisé en ce que** la streptavidine ou/et l'avidine sont fixées sur une phase solide et le récepteur glycane est détecté ou/et isolé sur sa fixation sur la phase solide.

31. Kit de réactif pour la détection ou/et l'isolation d'hydrate de carbone, comprenant :
(a) un composé selon l'une des revendications 1 à 10,
(b) une phase solide revêtue de streptavidine ou/et d'avidine.

32. Kit de réactif selon la revendication 31, comprenant de plus :
(c) au moins une enzyme séparant l'hydrate de carbone.

33. Kit de réactif pour la détection ou/et l'isolation de récepteur de glycane, comprenant :
(a) un composé selon l'une des revendications 1 à 10 qui peuvent éventuellement se présenter sous forme d'un conjugué selon l'une des revendications 13 à 15, et
(b) une phase solide revêtue de streptavidine ou/et d'avidine.

34. Composé de la formule (III) : 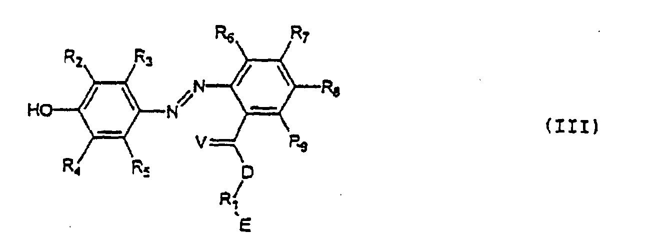 dans laquelle V, D et R₁ jusqu'à R₉ sont définis comme dans la revendication 6, et E signifie OH, SH, NH₂ ou COOH.

35. Utilisation d'un composé selon la revendication 34 pour la préparation de composé de la formule (II).
